# EUROPÄISCHE PATENTSCHRIFT

(11) **EP 1 517 911 B1**
(45) Veröffentlichungstag und Bekanntmachung des Hinweises auf die Patenterteilung: **29.07.2009**
(21) Anmeldenummer: 03735630.0
(22) Anmeldetag: 16.06.2003
(51) Int. Cl.: C07F 15/00, A61K 31/28, A61P 35/00

(54) **TUMORHEMMENDE PLATIN(II)-OXALATO-KOMPLEXE**
TUMOUR-INHIBITING PLATINUM (II) OXALATE COMPLEXES
COMPLEXES PLATINE(II)-OXALATO INHIBITEURS DE TUMEURS

(30) Priorität: 14.06.2002 DE 10226592
(43) Veröffentlichungstag der Anmeldung: 30.03.2005
(73) Patentinhaber: Keppler, Prof. Dr. Dr., Bernhard K., 2191 Gaweinstal (AT)
(72) Erfinder: KEPPLER, Bernhard, 68766 Hockenheim (DE)
(74) Vertreter: Kopecky, Helmut
(86) Internationale Anmeldenummer: PCT/EP2003/006323
(87) Internationale Veröffentlichungsnummer: WO 2003/106469

(56) Entgegenhaltungen:
- EP-A- 0 310 260
- EP-A- 0 464 210
- EP-A- 0 801 070
- US-A- 4 291 027
- US-A- 4 359 425
- US-A- 4 670 458
- PATENT ABSTRACTS OF JAPAN vol. 010, no. 194 (C-358), 8. Juli 1986 (1986-07-08) & JP 61 037794 A (MITSUI PETROCHEM IND LTD;OTHERS: 01), 22. Februar 1986 (1986-02-22) in der Anmeldung erwähnt

## Beschreibung

Die Erfindung betrifft tumorhemmende Platin(II)-oxalato-Komplexe und deren Verwendung als therapeutisches Mittel, insbesondere als tumorhemmendes Arzneimittel.

Es ist bekannt, dass einige Platin-Komplexe tumorhemmende Wirkungen haben. Neben Cisplatin stellen Carboplatin und Oxaliplatin mit der folgenden Struktur 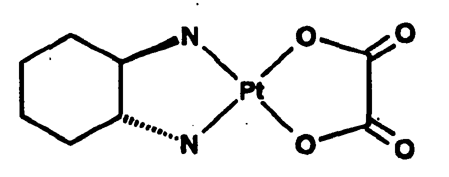 die beiden wichtigsten bekannten Plafinwirkstofle zur Tumorbekämpfung dar, wobei in Oxaliplatin die Abgangsgruppe des Komplexes bei seiner Verwendung als Antitumormittel Oxalat ist. Insbesondere hat jedoch Cisplatin ernsthafte Nebenwirkungen, wie Nierentoxizität, Myelosuppression und Ototoxizität.

Daher hat besonders Oxaliplatin, mit einem bidentaten *trans*-*R*,*R*-1,2-Ciaminocyclohexanliganden und einem ebenso bidentaten Oxalatoliganden mit einem von den anderen beiden Platin(II)-Komplexen abweichenden Wirkungsspektrum interessante Eigenschaften als Krebstherapeutikum. Vorteilhaft an der Verwendung von Oxatiplatin als Antitumormittel ist besonders dessen geringere Toxizität im Vergleich zu Cisplatin. Allerdings hat Oxaliplatin andere unerwünschte Nebenwirkungen, wie starke Neuropathien.

Zur Modifikation dieses Grundgerüsts sowie der Komplexeigenschaften wurde bisher die Abgangsgruppe des Komplexes saniert So sind in US 4,168,846 *Cis*Platin(II)-Komplexe mit *trans*-L-1,2-Diaminocyclohexan als Ligand ohne Substituenten am Cyclohexylring beschrieben, wobei die Komplexe als Abgangsgruppe neben Oxalat Malonat und substituierte Derivate der Malonsäure haben.

Die Verwendung von höheren Dicarbonsäuren bzw. Dicarbonsäurederivaten als Liganden in Platin(II)-Komplexen, wie Malonat oder Succinat, ist beispielsweise auch in JP 61037794 beschrieben.

Des weiteren ist eine Reihe von Platin(II)-Komplexen mit monodentaten Anionen, wie Pyrophosphat, in US 4,234,500 beschrieben.

Auch sind Platin(II)-Komplexe bekannt, die einen modifizierten Diaminocyclohexanliganden haben. Beispielsweise sind in US 4,359,425 Platin(II)-Komplexe beschrieben, die einen speziellen Diaminocyclohexanliganden haben, der bicyclisch und an den beiden den Aminogruppen benachbarten Kohlenstoffatomen durch eine (CH₂)ₙ-Brücke mit n = 1 oder 2 verbrückt ist In US 4,670,458 werden dihydroxylierte Diaminocyclohexanderivate als Dianiinocyclohexanliganden offenbart.

Außerdem sind in US 4,291,027 substituierte Cyclohexanderivate beschrieben, die Pyrophosphat als Abgangsgruppe haben.

Der vorliegenden Erfindung liegt die Aufgabe zugrunde, weitere tumorhemmende Platin(II)-oxalato-Komplexe zur Verfügung zu stellen.

Die Lösung der Aufgabe sind Verbindungen der allgemeinen Formel (I), 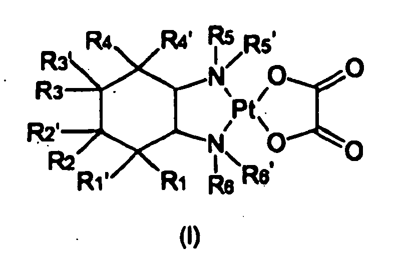 wobei die Substituenten R¹, R^{1'}, R², R^{2'}, R³, R^{3'}, R⁴, und R^{4'} unabhängig voneinander ausgewählt sind aus der Gruppe, bestehend aus Wasserstoff, verzweigten oder unverzweigten Alkyl-, verzweigten oder unverzweigten Alkenyl-, Cycloalkyl Cycloalkenyl-, Aryl- und Alkylarylresten,
die Substituenten R⁵, R^{5'}, R⁶, und R^{6'} unabhängig voneinander ausgewählt sind aus der Gruppe, bestehend aus Wasserstoff, verzweigten oder unverzweigten Alkyl-, und verzweigten oder unverzweigten Alkenylresten,
wobei gegebenenfalls jeweils wenigstens zwei der Substituenten R¹, R^{1'}, R², R^{2'}, R³, R^{3'}, R⁴, und R^{4'} miteinander wenigstens einen Alkylen-, Alkenylenrest oder einen unsubstituierten oder substituierten aromatischen Ring bilden können, und
wobei gegebenenfalls wenigstens eines der die Substituenten R¹, R^{1'}, R², R^{2'}, R³, R^{3'}, R⁴, und R^{4'} tragenden Kohlenstoffatome des Cyclohexanrings durch ein Heteroatom ersetzt ist, und
falls das Heteroatom Sauerstoff ist, die Substituenten R¹, R^{1'}, R². R^{2'}, R³, R^{3'}, R⁴, und/oder R^{4'} zusätzlich Hydroxyreste sein können, und
pharmazeutisch verträglich Salze davon,
mit der Maßgabe, dass
mindestens einer der Substituenten R¹, R^{1'}, R², R^{2'}, R³, R^{3'}, R⁴, oder R^{4'} ungleich Wasserstoff ist und die Reste R¹ oder R^{1'} und R⁴ oder R^{4'} miteinander keinen unsubstituierten C₁₋₂-Alkylenrest bilden.

Vorzugsweise sind die Substituenten R¹, R^{1'}, R², R^{2'}, R³, R^{3'}, R⁴, und R^{4'} unabhängig voneinander ausgewählt aus der Gruppe, bestehend aus Wasserstoff, C₁-C₁₅-Alkyl-, C₂-C₁₅-Alkonyl-, C₃-C₁₅-Cycloalkyl-, C₃-C₁₅-Cycloalkonyl-, C₆-C₁₄-Aryl- und C₁-C₁₅-Alkyl-C₆-C₁₄-Arylresten.

Besonders bevorzugt sind die Substituenten R¹, R^{1'}, R², R^{2'}, R³, R^{3'}, R⁴, und R^{4'} unabhängig voneinander ausgewählt aus der Gruppe, bestehend aus Wasserstoff, C₁-C₁₀-Alkyl-, C₂-C₁₀-Alkenyl-, C₃-C₁₀-Cycloalkyl-, C₃-C₁₀-Cycloalkenyl-, C₆-C₁₀-Aryl-und C₁-C₁₀-Alkyl-C₆-C₁₀-Arylresten, insbesondere sind die Substituenten R¹, R^{1'}, R², R^{2'}, R³, R^{3'}, R⁴, und R^{4'} ausgewählt aus der Gruppe, bestehend aus Wasserstoff, C₁-C₆-Alkyl-, C₂-C₆-Alkonyl-, C₃-C₆-Cycloalkyl-, C₃-C₆-Cycloalkenyl-, C₆-C₁₀-Aryl- und C₁-C₆-Alkyl-C₆-C₁₀-Arylresten.

Insbesondere bevorzugt sind die Substituenten R¹, R^{1'}, R², R^{2'}, R³, R^{3'}, R⁴, R^{4'} ausgewählt aus der Gruppe, bestehend aus Wasserstoff, C₁₋₁₀-Alkylresten, insbesondere Methyl-, Ethyl-, n-Propyl, i-Propyl, n-Butyl-, verzweigten Butylresten, n-Pentyl-, verzweigten Pentylresten, n-Hexyl- und verzweigten Hexylresten, C₃₋₁₀-Cycloalkylresten, insbesondere Cyclopropyl-, Cyclobutyl-, Cyclopentyl- und Cyclohexylresten, und C₆-C₁₄-Arylresten, insbesondere Phenylresten.

Die beiden am selben Kohlenstoffatom befindlichen Substituenten R¹, R^{1'}, R², R^{2'}, R³, R^{3'}, oder R⁴, R^{4'} können gleich oder verschieden sein.

Vorzugsweise sind ein bis drei, bevorzugt ein oder zwei und insbesondere einer der Substituenten R¹, R^{1'}, R², R^{2'}, R³, R^{3'}, R⁴, und R^{4'} ausgewählt aus der Gruppe, bestehend aus Alkyl-, Alkenyl Cycloalkyl-, Cycloalkenyl-, Aryl- und Alkylarylresten und die übrigen Reste sind Wasserstoffatome. Die Reste sind bevorzugt, wie vorstehend beschrieben.

In einer besonders bevorzugten Ausführungsform sind die Substituenten R¹, R^{1'}, R³, R^{3'}, R⁴, und R^{4'} gleich Wasserstoff und R² und R^{2'} sind ausgewählt aus der Gruppe, bestehend aus Wasserstoff, C₁-C₆-Alkyl-, C₂-C₆-Alkenyl-, C₃-C₆-Cycloalkyl-, C₃-C₆-Cycloalkenyl- und C₆-C₁₀-Arylresten und mit der Maßgabe, dass R² und R^{2'} nicht gleichzeitig Wasserstoff sind.

Ganz besonders bevorzugt sind Verbindungen, wobei die Substituenten R¹, R^{1'}, R³, R^{3'}, R⁴, und R^{4'} gleich Wasserstoff sind und die Substituenten R² oder R^{2'} ein C₁-C₆-Alkylrest, insbesondere Methyl-, Ethyl-, Propyl- oder t-Butylrest, oder ein C₆-C₁₀-Arylrest, insbesondere Phenylrest, sind, wobei gegebenenfalls der jeweils andere Rest R² oder R^{2'} Wasserstoff ist.

Ebenso ganz besonders bevorzugt sind Verbindungen, wobei die Substituenten R¹, R^{1'}, R³, R^{3'}, R⁴, und R^{4'} gleich Wasserstoff sind und R² oder R^{2'} ein C₁-C₆-Alkylrest, insbesondere Methyl-, Ethyl-, Propyl- oder t-Butylrest, oder ein C₆-C₁₀-Arylrest, insbesondere Phenylrest, sind, wobei entweder R² oder R^{2'} Wasserstoff sind.

In einer weiteren besonders bevorzugten Ausführungsform sind die Substituenten R¹, R^{1'}, R⁴, und R^{4'} gleich Wasserstoff, und R², R^{2'}, R³ und R^{3'} sind ausgewählt aus der Gruppe, bestehend aus Wasserstoff, C₁-C₆-Alkyl- und C₂-C₆-Alkenylresten, und mit der Maßgabe, dass R¹, R^{2'}, R³ und R^{3'} nicht gleichzeitig Wasserstoff sind.

Ganz besonders bevorzugt sind Verbindungen, wobei die Substituenten R¹, R^{1'}, R⁴, und R^{4'} gleich Wasserstoff sind, die Substituenten R², R^{2'}, R³ und R^{3'} ein C₁-C₆-Alkylrest sind, insbesondere Methyl-, Ethyl-, Propyl- oder t-Butylrest, wobei gegebenenfalls der jeweils andere Rest R² oder R^{2'} bzw. R³ oder R^{3'} Wasserstoff ist.

Ebenso ganz besonders bevorzugt sind Verbindungen, wobei die Substituenten R¹, R^{1'}, R⁴, und R^{4'} gleich Wasserstoff sind, R² oder R^{2'} und R³ oder R^{3'} ein C₁-C₆-Alkylrest, insbesondere ein Methyl-, Ethyl-, Propyl- oder t-Butylrest wobei R² oder R^{2'} und R³ oder R^{3'} Wasserstoff sind.

Wenn wenigstens zwei der Substituenten R¹, R¹, R², R^{2'}, R³, R^{3'}, R⁴, und R^{4'} miteinander wenigstens einen Alkylen-, Alkenylenrest oder einen Arylrest bilden, dann sind die diese bildenden bevorzugt C₁-C₁₅-Alkylen-, C₂-C₁₅-Alkenylenreste oder C₆-C₁₄-Arylreste. Dadurch werden bi-, tri- oder mehrcyclische Reste erhalten.

Es ist insbesondere bevorzugt, dass jeweils zwei an benachbarten Kohlenstoffatomen des Cyclohexanring befindliche Reste miteinander einen der vorstehend beschriebenen Reste bilden. Weiterhin ist insbesondere bevorzugt, dass ein C₆-C₁₄-Arylrest insbesondere ein Phenylrest, über die die Substituenten R², R^{2'} und R³, R^{3'} tragenden Kohlenstoffatome des Cyclohexanrings an den Cyclohexanring annelliert ist.

Es können auch zwei am selben Kohlenatom des Cyclohexanrings befindliche Reste miteinander einen der vorstehend beschriebenen Reste bilden, wobei die jeweiligen Spiroverbindungen erhalten werden. Besonders bevorzugt bilden nur jeweils zwei der Reste miteinander einen der vorstehend beschriebenen Reste, wobei ein bicyclisches System erhalten wird.

In einer bevorzugten Ausführungsform sind die Substituenten R⁵, R^{5'}, R⁶, und R^{6'} unabhängig voneinander ausgewählt sind aus der Gruppe, bestehend aus Wasserstoff, C₁-C₆-Alkyl-, oder C₂-C₆-Alkenylresten. Die C₁-C₆-Alkylreste sind bevorzugt Methyl-, Ethyl-, n-Propyl-, oder i-Propylreste. Die C₂-C₆-Alkenylreste sind bevorzugt Ethenyl oder Propenylreste. Weiterhin sind die Substituenten R⁵, R^{5'}, R⁶, und R^{6'} vorzugsweise Wasserstoff, Bevorzugt sind R⁵, R^{5'}, R⁶, und R^{6'} unsubstituiert.

Wenn wenigstens eines der die Substituenten R¹, R^{1'}, R², R^{2'}, R³, R^{3'}, R⁴, und R^{4'} tragenden Kohlenstoffatome des Cyclohexanrings durch ein Heteroatom ersetzt ist, ist das Heteroatom vorzugsweise ausgewählt aus der Gruppe, bestehend aus Sauerstoff, Stickstoff und Schwefel und es ist bevorzugt, dass ein bis drei, insbesondere ein oder zwei, besonders ein Heteroatom anwesend ist.

Falls eines der Kohlenstoffatome des Cyclohexanrings durch ein Heteroatom substituiert ist, so können in Abhängigkeit von der Bindigkeit des Heteroatoms weniger als zwei Substituenten an das Heteroatom gebunden sein. Sauerstoff und Schwefel sind z.B. unsubstituiert und Stickstoff ist entsprechend der Position im Heterocyclus einfach substituiert durch einen der Substituenten R¹, R^{1'}, R², R^{2'}, R³, R^{3'}, R⁴, oder R^{4'}.

Bevorzugt handelt es bei dem Heteroatom um Sauerstoff, der anstelle des R³ und R^{3'} oder R² und R^{2'} tragenden Kohlenstoffatums in den Cyclohexanring eingebaut worden ist, wobei mindestens ein verbleibender Substituent R¹, R^{1'}, R², R^{2'}, R³, R^{3'}, R⁴ oder R^{4'} ungleich Wasserstoff ist.

Falls das Heteroatom Sauerstoff ist, kann der entsprechend modifizierte Cyclohexanring ein Aminozucker, z.B. auf der Basis von Glucose 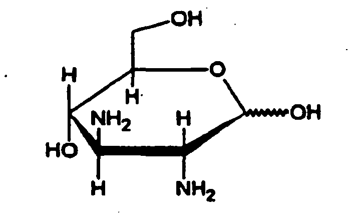 sein.

Die beiden Aminogruppen am Cyclohexanring in den erfindungsgemäßen Verbindungen können relativ zueinander eine *cis*- oder *trans*-Anordnung haben. Bevorzugt haben sie eine *trans*-Anordnung, Die beiden Kohlenstoffatome des Cyclohexanrings, die die Aminogruppen tragen, können eine R,R-, S,S-, R,S- oder S,R-Konfiguration haben.

Eine mögliche Anordnung der beiden Aminogruppen und des Heteroatoms Sauerstoff ist im folgenden dargestellt: 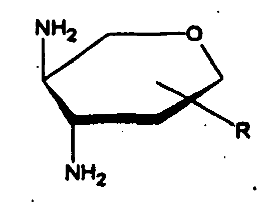 Weiterhin sind Verbindungen bevorzugt, bei denen die Substituenten R⁵, R^{5'}, R⁶ und R^{6'} Wasserstoff sind und gegebenenfalls wenigstens eines der die Substituenten R¹, R^{1'}, R², R^{2'}, R³, R^{3'}, R⁴, und R^{4'} tragenden Kohlenstoflatome des Cyclohexanrings durch ein Heteroatom ersetzt ist, und
falls das Heteroatom Sauerstoff ist, die Substituenten R¹, R^{1'}, R², R^{2'}, R³, R^{3'}, R⁴, und R^{4'} unabhängig voneinander ausgewählt sind aus der Gruppe, bestehend aus Wasserstoff, Alkyl-, Alkenyl-, Cycloalkyl-, Cycloalkenyl-, Aryl- und Alkylarylresten.

Im Sinne der vorliegenden Beschreibung umfassen die Begriffe "Alkyl" oder "Alkenyl" immer unverzweigtes oder verzweigtes "Alkyl" oder "Alkenyl".

Die physiologisch verträglichen Salze der erfindungsgemäßen Verbindungen sind vorzugsweise ausgewählt aus der Gruppe, bestehend aus organischen oder anorganischen Additionssalzen, die insbesondere mit den nachstehend beschriebenen Anionen und Kationen gebildet werden können.

Die Anionen sind vorzugsweise ausgewählt aus der Gruppe, bestehend aus Halogen, wie Chlorid und Bromid, Pseudohalogenen, Phosphat, Carbonat, Nitrat, Perchlorat, Sulfat, Citrat, Lactat, Tartrat, Maleat, Fumarat, Mandelat, Benzoat, Ascorbat, Cinnamat, Glycollat, Methansulfonat Formiat, Malonat, Naphthalin-2-sulfonat, Salicylat und Acetat.

Die Kationen sind vorzugsweise ausgewählt aus der Gruppe, bestehend aus H+, Natrium- und Kalium-Kationen.

Die erfindungsgomäßen Verbindungen haben eine überraschend gute tumorhemmende Wirkung.

Die Synthese der efindungsgemäßen Verbindungen erfolgt nach an sich bekannten Verfahren beispielsweise ausgehend von K₂[PtCl₄], wobei die beiden bidentaten Liganden sukzessive eingeführt werden. Dazu kann zuerst das Tetrachloroplatinat(II) mit dem geeignet substituierten Diamin umgesetzt werden. Der erhaltene Diamin(dichloro)platin(II)-Kompfex kann dann durch Umsetzung mit Silbernitrat oder Silbersulfat aktiviert werden. Die so gewonnene Diaqua(diamin)- oder Aqua(sulfato)diamin-Verbindung kann dann durch Ligandenaustausch mit Oxalsäure oder Natriumoxalat in die entsprechende erfindungsgemäße Verbindung überführt werden. Beispielsweise kann (*SP*-4-3)-(*trans-*Cyclohexan-1,2-diemin-4-methyl)-oxalatoplatin(II) wie vorstehend beschrieben hergestellt werden.

Die nachstehenden Schemata zeigen am Beispiel des 4-Methyl-substituierten Derivats mögliche Synthesewege zur Herstellung der erfindungsgemäßen Verbindungen.

Zur Herstellung von (*SP*-4-3)-(*trans*-Cyclohexan-1,2-diamin-4-methyl)-oxalatoplatin(II) können zunächst isomere Gemische von trans-4-Methylcyclohexan-1,2-diamine ausgehend von 4-Methylcyclohexanon gemäß Schema 1 hergestellt werden. Wie dem Schema 1 zu entnehmen, wird dazu nach der Bromierung und Umsetzung mit Hydroxylamin, das dabei erhaltene Dioxim mit Natrium in absolutem Ethanol reduziert.

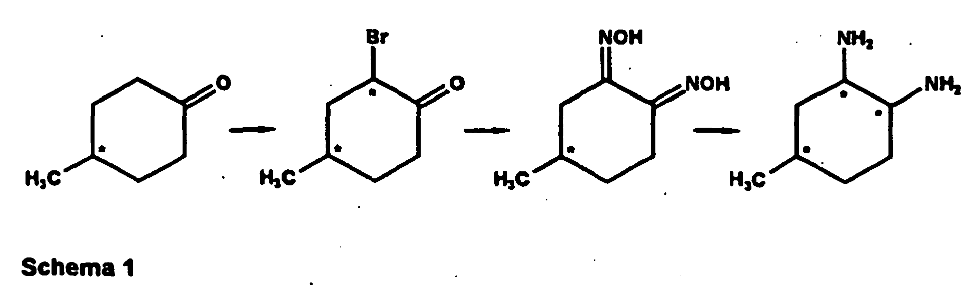

Zur Herstellung der übrigen erfindungsgemäßen Verbindungen kann das Cyclohexanon in Schema 1 auch durch die Substituenten R¹, R^{1'}, R², R^{2'}, R³, R^{3'}, R⁴ und/oder R^{4'} substituiert sein, wie vorstehend beschrieben. Insbesondere kann auch das entsprechende Derivat mit einem Ethylrest statt eines Methylrests analog hergestellt werden. Alternativ kann anstelle des substituierten Cyclohexanons auch ein geeignet substituiertes Cyclohexan-1,2-dion-Derivat verwendet werden, das dann wie zuvor beschrieben direkt durch Umsetzung mit Hydroxylamin in das entsprechende Oxim überführt werden kann.

Dabei werden isomere Gemische von substituiertem trans-Cyclohexan-1,2-diamin erhalten.

Das substituierte Cyclohexanol oder Cyciohexan-1,2-dion kann auch durch die Substituenten R¹, R^{1'}, R², R^{2'}, R³, R^{3'}, R⁴ und/oder R^{4'} substituiert sein, wie vorstehend beschrieben.

Alternativ wird das geeignet substituierte Cyclohexanonderivat zuerst zum Alkohol reduziert und dieser dann zum Cycloalken dehydratisiert. Durch 1,2-Addition von Natriumazid an die C=C-Doppelbindung wird das korrespondierende Diazid synthetisiert und dieses im Anschluss mittels Wasserstoff am Lindlar-Katalysator zum Diamin reduziert.

Die nach der Umsetzung der vorstehend beschriebenen Diaminocyclohexanliganden mit K₂[PtCl₄] erhaltenen Diamin(dichloro)platin(II)-Verbindungen können dann in die entsprechenden Diaqua(diamin)- oder Aqua(sulfato)-Verbindungen jeweils unter Verwendung von Silbernitrat oder Silbersulfat umgewandelt werden. Nach der Zugabe von Oxalsäure oder Natriumoxalat werden die erfindungsgemäßen Verbindungen als isomere Gemische erhalten, wie beispielsweise im nachstehenden Schema 2 gezeigt.

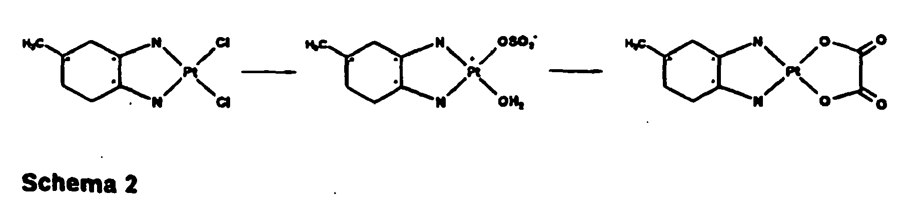

Statt des Methylsubstituenten können auch die Substituenten R¹, R^{1'}, R², R^{2'}, R³, R^{3'}, R⁴ und/oder R^{4'} anwesend sein, wie vorstehend beschrieben,

Nach der Herstellung der erfindungsgemäßen Verbindungen als isomere Gemische können diese in die reinen Isomere nach bekannten Verfahren getrennt werden. Die Racematspaltung erfolgt vorzugsweise auf der Stufe der Diamine nach an sich bekannten Verfahren wie Bildung diastereomerer Salze mit chiralen Säuren oder Säulenchromatographie an chiraler stationärer Phase.

Die Erfindung betrifft weiterhin Verbindungen der Formel (I) als therapeutisches oder prophylaktisches Mittel. Außerdem betrifft die Erfindung die Verwendung einer Verbindung der Formel (I) zur Herstellung eines Medikaments zur Bekämpfung und/oder Prophylaxe von Tumorerkrankungen.

Ferner wird die Aufgabe der vorliegenden Erfindung durch ein Arzneimittel, insbesondere zur Therapie und/oder Prophylaxe von Tumorerkrankungen, gelöst, das die erfindungsgemäße Verbindung umfasst. Die erfindungsgemäße Verbindung kann unter anderem zur Prophylaxe und/oder Behandlung von Krebserkrankungen eingesetzt werden, wie

Im folgenden wird das Arzneimittel, enthaltend eine erfindungsgemäße Verbindung, genauer beschrieben.

Das erfindungsgemäße Arzneimittel wird vor allem intravenös, aber auch intramuskulär, intraperitoneal, subkutan oder peroral verabreicht. Auch eine außerliche Applikation ist möglich. Bevorzugt ist die Verabreichung durch intravenöse Injektion oder intravenöse Infusion.

Das Arzneimittel wird nach an sich bekannten Verfahren hergestellt, wobei die erfindungsgemäße Verbindung als solche öder gegebenenfalls in Kombination mit geeigneten pharmazeutischen Trägerstoffen eingesetzt wird. Enthält das erfindungsgemäße Arzneimittel neben dem Wirkstoff pharmazeutische Trägerstoffe, beträgt der Wirkstoffgehalt dieser Mischung 0,1 bis 99,5, vorzugsweise 0,5 bis 95 Gew.-% der Gesamtmischung.

Das erfindungsgemäße Arzneimittel kann in jeder geeigneten Formulierung angewandt werden unter der Voraussetzung, dass die Ausbildung bzw. Aufrechterhaltung von ausreichenden Wirkstoffpegeln gewährleistet ist. Das kann beispielsweise durch orale oder parenterale Gabe in geeigneten Dosen erreicht werden. Vorteilhafterweise liegt die pharmazeutische Zubereitung des Wrkstoffs in Form von Einheitsdosen vor, die auf die gewünschte Verabreichung abgestimmt sind. Eine Einheitsdosis kann zum Beispiel eine Tablette, ein Drageé, eine Kapsel, ein Suppositorium oder eine gemessene Volumenmenge eines Pulvers, eines Granulates, einer Lösung, einer Emulsion oder einer Suspension sein.

Unter "Einheitsdosis" im Sinne der vorliegenden Erfindung wird eine physikalisch bestimmte Einheit verstanden, die eine individuelle Menge des aktiven Bestandteils in Kombination mit einem pharmazeutischen Trägerstoff enthält und deren Wirkstoffgehalt einem Bruchteil oder Vielfachen einer therapeutischen Einzeldosis entspricht. Eine Einzeldosis enthält vorzugsweise die Menge Wirkstoff, die bei einer Applikation verabreicht wird und die gewöhnlich einer ganzen, einer halben, einer drittel oder einer viertel Tagesdosis entspricht. Wenn für eine einzelne therapeutische Verabreichung nur ein Bruchteil, wie die Hälfte oder ein Viertel der Einheitsdosis benötigt wird, ist die Einheitsdosis vorteilhafterweise teilbar, z.B. in Form einer Tablette mit Bruchkerbe.

Die erfindungsgemäßen Arzneimittel können, wenn sie in Einheitsdosen vortiegen und für Applikationen z.B. am Menschen bestimmt sind, etwa 0,1 bis 500 mg, bevorzugt 10 bis 200 mg und insbesondere 50 bis 150 mg Wirkstoff enthalten.

Im allgemeinen werden in der Humanmedizin der oder die Wirkstoffe in einer Tagesdosis von 0,1 bis 5, vorzugsweise 1 bis 3 mg/kg Körpergewicht, gegebenenfalls in Form mehrerer, vorzugsweise 1 bis 3 Einzelgaben zur Erzielung der gewünschten Ergebnisse verabreicht. Eine Einzelgabe enthält den oder die Wirkstoffe in Mengen von 0,1 bis 5, vorzugsweise 1 bis 3 mg/kg Körpergewicht.

Bei einer oralen Behandlung können ähnliche Dosierungen zur Anwendung kommen.

Die therapeutische Verabreichung des erfindungsgemäßen Arzneimittels kann 1 bis 4 mal am Tage zu festgelegten oder variierenden Zeitpunkten erfolgen, z.B. jeweils vor den Mahlzeiten und/oder am Abend. Es kann jedoch erforderlich sein, von den genannten Dosierungen abzuweichen, und zwar in Abhängigkeit von der Art, dem Körpergewicht und dem Alter der zu behandelnden Individuen, der Art und Schwere der Erkrankung, der Art der Zubereitung und der Applikation der Arzneimittel sowie dem Zeitraum bzw. Intervall, innerhalb welchem die Verabreichung erfolgt So kann es in einigen Fallen ausreichend sein, mit weniger als der oben genannten Menge Wirkstoff auszukommen, während in anderen Fällen die oben angeführte Wirkstoffmenge überschritten werden muss. Es kann sich auch als zweckmäßig erweisen, die Arzneimittel nur einmalig oder im Abstand von mehreren Tagen zu verabreichen.

Die Festlegung der erforderlichen optimalen Dosierung und Applikationsart der Wirkstoffe kann durch jeden Fachmann aufgrund seines Fachwissens erfolgen.

Die erfindungsgemäßen Arzneimittel bestehen in der Regel aus den erfindungsgemäßen Verbindungen und nichttoxischen, pharmazeutisch verträglichen Arzneimittelträgem, die als Zumischung oder Verdünnungsmittel, beispielsweise in fester, halbfester oder flüssiger Form oder als Umhüllungsmittel, beispielsweise in Form einer Kapsel, eines Tablettenüberzugs, eines Beutels oder eines anderen Behältnisses für den therapeutisch aktiven Bestandteil in Anwendung kommen. Ein Tragerstoff kann z.B. als Vermittler für die Arzneimittelaufnahme durch den Körper, als Formulierungshilllsmittel, als Süßungsmittel, als Geschmackskonigens, als Farbstoff oder als Konservierungsmittel dienen.

Zur oralen Anwendung können z.B. Tabletten Drageés, harte und weiche Kapseln, z.B. aus Gelatine, dispergierbare Pulver, Granulate, wässrige und ölige Suspensionen, Emulsionen, Lösungen oder Sirupe kommen.

Tabletten können inerte Verdünnungsmittel, z.B. Calciumcarbonat, Calciumphosphat, Natriumphosphat oder Laktose; Granulierungs- und Verteilungsmittel, z.B. Maisstärke oder Alginate; Bindemittel, z.B. Stärke, Gelatine oder Akaziengummi; und Gleitmittel, z.B. Aluminium- oder Magnesiumstearat, Talkum oder Silikonöl, enthalten. Sie können zusätzlich mit einem Überzug versehen sein, der auch so beschaffen sein kann, dass er eine verzögerte Auflösung und Resorption der Arzneimittelzubereitung im Gastrointestinaltrakt bewirkt, so dass z.B. eine bessere Verträglichkeit, Protahierung oder Retardierung erreicht wird. Gelatinekapseln können den Arzneistoff vermischt mit einem festen, z.B. Calciumcarbonat oder Kaolin, oder einem öligen, z.B. Oliven-, Erdnuss-, oder Paraffinöl, Verdünnungsmittel enthalten.

Wässrige Suspensionen können Suspendiermittel, z.B. Natriumcarboxymethylcellulose, Methylcellulose, Hydroxypropylcellulose, Natriumalginat, Polyvinylpyrrolidon, Traganthgummi oder Akaziengummi; Dispergier- und Benetzungsmittel, z.B. Polyoxyethylenstearat, Heptadecaethylenoxycatanol, Polyoxyethylensorbitolmonooleat oder Lecithin; Konservierungsmittel, z.B. Methyl- oder Propylhydroxybenzoate; Geschmacksmittel; Süßungsmittel, z.B. Saccharose, Laktose, Natriumcyclamat, Dextrose, Invertzuckersirup, enthalten.

Ölige Suspensionen können z.B. Erdnuss-, Oliven-, Sesam-, Kokos- oder Paraffinöl und Verdickungsmittel, wie z.B. Bienenwachs, Hartparaffin oder Cetylalkohol, enthalten; ferner Süßungsmittel, Geschmacksmittel und Antioxidantien.

In Wasser dispergierbare Pulver und Granulate können die erfindungsgemäße Verbindung in Mischung mit Dispergier-, Benetzungs- und Suspendiermitteln, z.B. den oben genannten, sowie mit Süßungsmitteln, Geschmacksmitteln und Farbstoffen enthalten.

Emulsionen können z.B. Oliven-, Erdnuss-, oder Paraffinöl neben Emulgiermitteln, wie z.B. Akaziengummi, Traganthgummi, Phosphatiden, Sorbitanmonooleat, Polyoxyethylensorbitanmonooleat, und Süßungs- und Geschmacksmittel enthalten.

Wässrige Lösungen können Konservierungsmittel, z.B. Methyl- oder Propylhydroxybenzoate; Verdickungsmittel; Geschmacksmittel; Süßungsmittel, z.B. Saccharose, Laktose, Natriurncyclamat Dextrose, Invertzuckersirup, sowie Geschmacksmittel und Farbstoffe enthalten.

Zur parenteralen Anwendung der Arzneistoffe dienen steril injizierbare, wässrige Lösungen, isotonische Satzlösungen oder sonstige Lösungen.

Die vorliegenden Erfindung betrifft auch ein Verfahren zur prophylaktischen und/oder therapeutischen Behandlung eines Säugetiers durch Verabreichen einer erfindungsgemäßen Verbindung, das einer solchen Behandlung bedarf. Das Säugetier ist ausgewählt aus der Gruppe bestehend aus Menschen und Tieren. Bevorzugt bedarf das Säugetier einer Behandlung gegen eine Tumorerkrankung, wie vorstehend beschrieben.

Die Erfindung wird nunmehr anhand von Beispielen näher erläutert.

### Synthesebeispiel 1

### 2-Brom-4-methyl-cyclohexanon

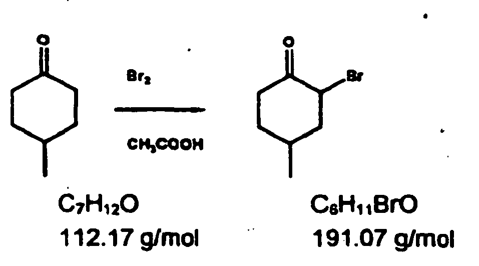

| | |
|---|---|
| Ansatz: | 50 g = 0.446 mmol 4-Methylcyclohexanon |
| | 74 g = 0.463 mmol Brom |
| | 92 ml 99%ige Essigsäure |

50 g 4-Methylcyclohexanon werden in einem Zweihalskolben mit Tropftrichter und Liebigkühler mit 120 ml Wasser, 92 ml 99%ige Essigsäure und 2 Tropfen Brom versetzt. Die Reaktionsmischung wird auf ca. 50°C erwärmt bis die Reaktion startet. Dann werden 74 g Brom über den Zeitraum von 2 Stunden so langsam zugetropft, dass die Temperatur bei 35 - 40 °C bleibt. Danach wird die Lösung mit festem Na₂CO₃ neutralisiert, so dass das Bromketon als gelbe ölige Flüssigkeit abgeschieden wird. Die organische Phase wird mit verdünnter Na₂CO₃ - Lösung und anschließend mit Wasser gewaschen. Das Produkt wird mit Na₂SO₄ getrocknet und filtriert.

| | |
|---|---|
| Aussehen: | gelbe ölige Flüssigkeit |
| Ausbeute: | 33.3 g = 0.174 mol = 39% (Literatur: 78%) |

α-Bromketone zersetzten sich sehr leicht bei Lagerung oder Erhitzten, daher wird auf eine Reinigung und Charakterisierung verzichtet. Nebenprodukte beeinflussen die Isolierung des Dioxims nicht.

### Synthesebeispiel 2

### 4-Methylcyclohexan-1,2-dioxim

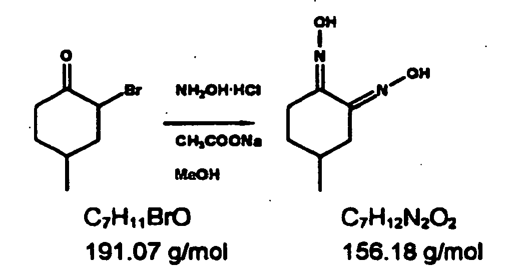

| | |
|---|---|
| Ansatz: | 33.2 g = 0.174 mol 2-Brom-4-methyl-cyclohexanon |
| | 70.4 g = 1.01 mol Hydroxylaminhydrochlorid |
| | 145.3 g Natriumacetat Trihydrat |
| | 126 ml Methanol |

70.4 g Hydroxylaminhydrochlorid und 145.3 g Natriumacetat Trihydrat werden in 126 ml Methanol und 146 ml Wasser zum Sieden erhitzt. 33.2 g 2-Brom-4-methyl-cyclohexanon werden in einem Zeitraum von 1.5 Stunden zu der siedenden Lösung getropft. Anschließend wird noch 1 Stunde lang unter Rückfluß gekocht und dann ca. 100 ml Methanol abdestilliert bis die zurückbleibende Lösung trüb wird. Der Rückstand wird auf Raumtemperatur abgekühlt und dreimal mit je 80 ml Benzen ausgeschüttelt. Die vereinigten Benzenphasen werden mit ca. 500 ml Petrolether (Kp. 40-60°C) versetzt und 2 Tage bei 4°C gekühlt. Am Boden setzt sich ein braunes Öl ab, das nach Abdekantieren der überstehenden Lösung, in Wasser umkristallisiert wird. Durch Einsatz einer kontinuierlichen Extraktionsapparatur konnte die Ausbeute bei weiteren Ansätzen auf 14% gesteigert werden.

| | |
|---|---|
| Aussehen: | weißer Feststoff |
| Ausbeute: | 2 g = 0.013 mol = 7.5% (Literatur 25%) |

### NMR-Spektren:

**¹H-NMR-Spektrum** in Methanol-d4:
δ = 1.06 [d, 3H, C*H*₃, ³J_{H,H} = 6.53 Hz], 1.26 [m, 1H], 1.74 [m, 1H], 1.82 [m, 1H], 1.98 [m, 1H], 2.37 [m, 1H], 2.97 [m, 2H].

**¹³C-NMR-Spektrum** in Methanol-d4:
δ = 20.8 [*C*H₃], 23.9 [*C*H₂], 29.2 [*C*H], 30.4 [*C*H₂], 33.0 [*C*H₂], 153.1 [*C*=NOH], 153.1 [*C*=NOH].

### Synthesebeispiel 3

### 4-Methyl-trans-cyclohexan-1,2-diamindihydrogensulfat

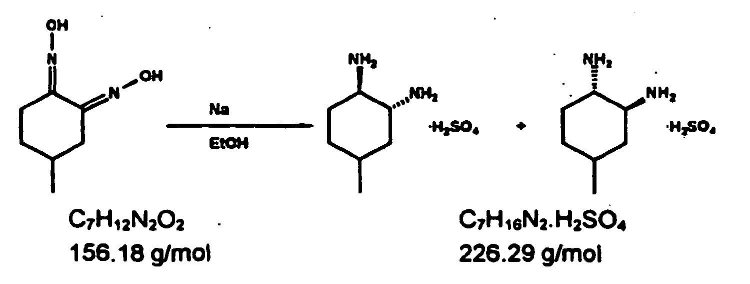

| | |
|---|---|
| Ansatz: | 4 g = 0.026 mol 4-Methylcyclohexan-1,2-dioxim |
| | 30 g = 1.305 mol Natrium |
| | 100 ml Ethanol absolut |

4 g 4-Methylcyclohexan-1,2-dioxim werden in einem 1l Dreihalskolben mit Rückflußkühler unter N₂ - Atmosphäre in 100 ml absolutem Ethanol gelöst und zum Sieden erhitzt. 30 g Natrium wird in kleinen Stücken zugegeben, so dass schließlich eine große an der Flüssigkeitsoberfläche schwimmende Kugel aus geschmolzenem Natrium gebildet wird. Ein Überzug aus Natriumethanolat an der Oberfläche der Kugel wird durch weitere Zugabe von absolutem Ethanol entfernt. Die Reaktionsmischung wird so lange gekocht bis alles Natrium gelöst ist. Die Lösung wird mit Wasserdampf destilliert, um zuerst den Alkohol zu entfernen, und dann das Amin zu erhalten. Das Ende der Destillation ist erreicht, wenn das Destillat nicht mehr alkalisch ist. Die aminhaltige Fraktion wird mit 3 M Schwefelsäure angesäuert und am Rotationsverdampfer bis zur Trockene eingedampft. Das rosafarbene Rohprodukt wird mit 40 ml Ethanol unter Rühren auf 50°C erhitzt, das rosa Nebenprodukt löst sich und das weiße 4-Methyl-*trans-*cyclohexan-1,2-diamindihydrogensulfat wird über einen G4-Filtertiegel abgesaugt und an der Luft getrocknet.

| | | | | | |
|---|---|---|---|---|---|
| Aussehen: | | weißer Feststoff | | | |
| Ausbeute: | | 3.067 g = 0.013 mol = 52% = (Literatur 78%) | | | |
| | | | | | |
| Elementaranalyse: | | berechnet für C₇H₁₆N₂· H₂SO₄ | | | 226.29 g/mol |
| | C | H | | N O S | |
| berechnet | 37.15 | 8.02 | 12.38 | 28.28 | 14.17 |
| gefunden | 37.27 | 7.80 | 12.17 | | |

| | | |
|---|---|---|
| **IR-Spektrum** [370-7000 cm⁻¹,Csl], charakteristische Banden (in cm⁻¹): | | |
| | im Bereich 2200-3300 | ν(N-H) |
| | 1651 | δ(N-H) |
| | 1550 | δ(N-H) |

### NMR-Spektren:

Es sind 2 Isomere nachweisbar. Das Isomere mit der geringeren Konzentration wird mit bezeichnet.

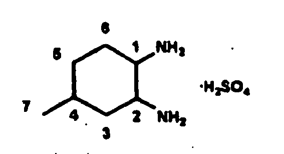

**¹H-NMR-Spektrum** in D₂O:
δ = 0.85 [d, 3H, H(7), ³J_{H,H} = 6.53 Hz], 0.90 [d, 3H, H(7'), ³J_{H,H} = 7.03 Hz], 0.99 [m, 1H, H(5)], 1.15 [m, 1H, H(3)], 1.42 - 1.55 [m, 2H, H(4), H(6)], 1.66 - 1.77 [m, 1H, H(5)], 1.99 - 2.10 [m, 2H, H(6) und H(3)], 3.23 - 3.39 [m, 2H, H(1) und H(2)], 3.50 - 360 [m, 2H, H(1') und H(2')].

**¹³C-NMR-Spektrum** in D₂O:
δ = 17.9 [C(7')], 20.7 [C(7)], 24.0 [C(6')], 25.8 [C(4')], 27.8 [C(5')], 29.5 (C(6)], 30.2 [C(4)], 31.5 [C(5)], 33.8 [C(3')], 37.6 [C(3)], 48.7 [C(1' oder 2')], 51.2 [C(1' oder 2')], 52.4 [C(1 oder 2)], 52.5 [C(1 oder 2)].

### Synthesebeispiel 4

### 2-Brom-4-ethyl-cyclohexanon

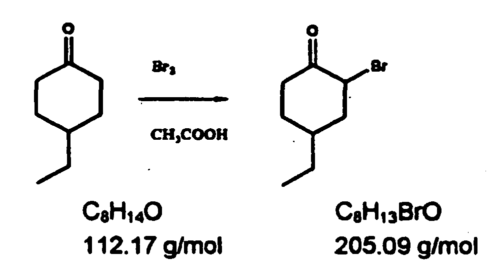

| | |
|---|---|
| Ansatz: | 46 g = 0.36 mmol 4-Ethylcydohexanon |
| | 66 g = 0.37 mmol Brom |
| | 82 ml 99%ige Essigsäure |

46 g 4-Ethylcyclohexanon werden in einem Zweihalskolben mit Tropftrichter und Liebigkühler mit 106 ml Wasser, 82 ml 99%ige Essigsäure und 2 Tropfen Brom versetzt. Die Reaktionsmischung wird auf ca. 50°C erwärmt bis die Reaktion startet Dann werden 59.2 g Brom über den Zeitraum von 1.5 Stunden so langsam zugetropft, dass die Temperatur bei 35 - 40 °C bleibt. Danach wird die Lösung mit festem NaCO₃ neutralisiert, sodass das Bromketon als gelbe ölige Flüssigkeit abgeschieden wird. Die organische Phase wird mit verdünnter Na-CO₃ - Lösung und anschließend mit Wasser gewaschen. Das Produkt wird über Na₂SO₄ filtriert.

| | |
|---|---|
| Aussehen: | gelbe Flüssigkeit |
| Ausbeute: | 54 g = 0.263 mol = 73% |

Das α-Bromketone zersetzten sich sehr leicht bei Lagerung oder Erhitzen, daher wird auf eine Reinigung und Charakterisierung verzichtet. Nebenprodukte beeinflussen die Isolierung des Dioxims nicht

### Synthesebeispiel 5

### 4-Ethyleyclohexan-1,2-dioxim

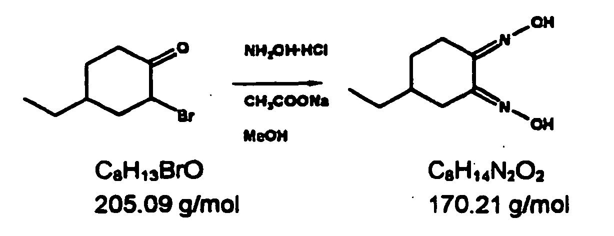

| | |
|---|---|
| Ansatz: | 54 g = 0.263 mol 2-Brom-4-ethyl-cyclohexanon |
| | 105.7 g = 1.52 mol Hydroxylaminhydrochlorid |
| | 212 g Natriumacetat Trihydrat |
| | 185 ml Methanol |

105.7 g Hydroxylaminhydrochlorid und 212 g Natriumacetat Trihydrat werden in 185 ml Methanol und 185 ml Wasser zum Sieden erhitzt. 54 g 1-Brom-4-ethylcyclohexanon werden in einem Zeitraum von einer Stunde zu der siedenden Lösung getropft. Anschließend wird noch 1 Stunde lang unter Rückfluß gekocht und dann destilliert bis die zurückbleibende Lösung trüb wird. Der Rückstand wird auf Raumtemperatur gekühlt und mit ca. 150 ml Benzen in einer kontinuierlichen Extraktionsapparatur extrahiert (in 4 Portionen a 100 ml, je 2 Stunden lang). Die Benzenlösung wird mit Petrolether (Kp. 40-60°C) auf ca. 900 ml aufgefüllt und über Nacht bei 4°C gekühlt. Am Boden setzt sich braunes Öl ab, das nach Abdekantieren der überstehenden Lösung in Wasser so oft umkristallisiert wird, bis das weiße 4-Ethylcyclohexan-1,2-dioxim erhalten wird (4 - 5-mal).

| | |
|---|---|
| Aussehen: | weißer plättchenförmiger Feststoff |
| Ausbeute: | 11.9 g = 0.0699 mol = 28.6% |

### NMR-Spektrein:

**¹H-NMR-Spektrum** in Methanol-d4:
δ = 0.98 [t, 3H, C*H*₃, ³J_{H,H} = 7.53 Hz], 1.25 [m, 1H], 1.40 [m, 2H, C*H*₂CH₃], 1.50 [m, 1H], 1.88 [m, 1H], 2.03 [m, 1H], 2.37 [m, 1H], 2.96 [m, 2H].

**¹³C-NMR-Spektrum** in Methanol-d4:
δ = 10.73 [*C*H₃], 23.9 [*C*H₂], 27.9 [*C*H₂], 28.8 [*C*H₂], 30.8 [*C*H₂], 35.9 [*C*H], 153.3 [*C*=NOH], 153.5 [*C*=NOH].

### Synthesebeispiel 6

### 4-Ethyl-trans-cyclohexan-1,2-diamindihydrogensulfat

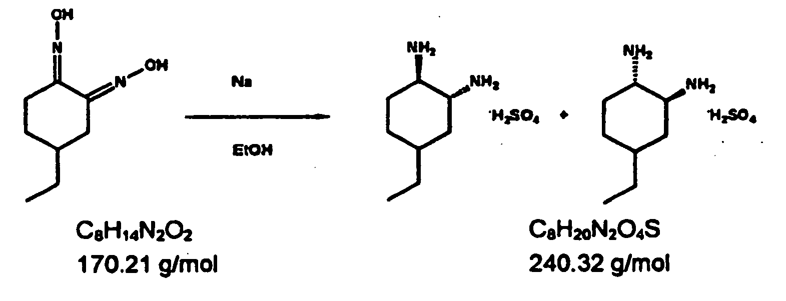

| | |
|---|---|
| Ansatz: | 9 g = 0.053 mol 4-Ethylcyclohexan-1,2-dioxim |
| | 50.5 g = 2.20 mol Natrium |
| | 280 ml Ethanol absolut |

9 g 4-Ethylcyclohexan-1,2-dioxim werden in einem 1l Dreihalskolben mit Rückflußkühler unter N₂-Atmosphäre in 100 ml absolutem Ethanol gelöst und zum Sieden erhitzt. 50.5 g Natrium wird in kleinen Stücken zugegeben, so dass schließlich eine große an der Flüssigkeitsoberfläche schwimmende Kugel aus geschmolzenen Natrium gebildet wird. Ein Überzug aus Natriumethanolat an der Oberfläche der Kugel wird durch weitere Zugabe von absolutem Ethanol entfernt. Die Reaktionsmischung wird so lange gekocht bis alles Natrium gelöst ist. Die Lösung wird mit Wasserdampf destilliert um zuerst den Alkohol zu entfernen und dann das Amin zu erhalten. Das Ende der Destillation ist erreicht, wenn das Destillat nicht mehr alkalisch ist. Die aminhaltige Fraktion wird mit 3 M Schwefelsäure angesäuert und am Rotationsverdampfer bis zur Trockene eingedampft. Das rosafarbene Rohprodukt wird mit 40 ml Ethanol unter Rühren auf 50°C erhitzt, das rosa Nebenprodukt löst sich und das weiße 4-Ethyl-*trans-*cyclohexan-1,2-diamindihydrogensulfat wird über einen G4-Filtertiegel abgesaugt und an der Luft getrocknet.

| | | | | | |
|---|---|---|---|---|---|
| Aussehen: | | weißer Feststoff | | | |
| Ausbeute: | | 3.055 g = 0.013 mol = 16 % | | | |
| | | | | | |
| Elementaranalyse: | | berechnet für C₈H₂₀N₂· H₂SO₄ | | | 240.32 g/mol |
| | C | H | N | O | S |
| berechnet | 39.98 | 8.39 | 11.68 | 26.63 | 13.34 |
| gefunden | 40.11 | 8.10 | 11.64 | | |

| | | |
|---|---|---|
| **IR-Spektrum** [370-7000 cm⁻¹,Csl], charakteristische Banden (in cm⁻¹): | | |
| | im Bereich 2200-3300 | ν(N-H) |
| | 1637 | δ(N-H) |
| | 1546 | δ(N-H) |

### Synthesebeispiel 7

### (SP-4-3)-Dichloro(4-methyl-trans-cyclohexan-1,2-diamin)platin(II)

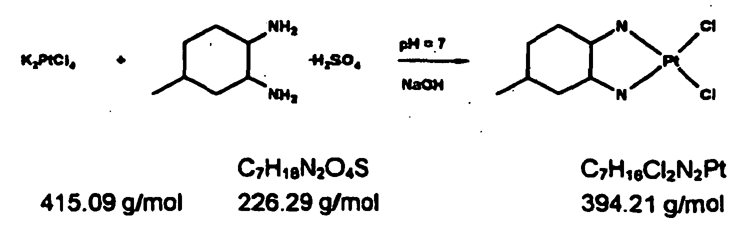

| | |
|---|---|
| Ansatz: | 2.15 g = 9.50 mmol 4-Methyl-*trans*-cyclohexan-1,2- |
| | diamindihydrogensulfat |
| | 3.944 g = 9.50 mmol K₂PtCl₄ |

3.944 g K₂PtCl₄ werden in 50 ml tridestilliertem Wasser gelöst und mit 2.15 g 4-Methyl-*trans*-cyclohexan-1,2-diamindihydrogensulfat suspendiert. Anschließend wird der pH-Wert mit 0.5 M NaOH auf 7 eingestellt und mit einem 718 Stat Titrino der Fa. Methrom mit 0.1 M NaOH auf diesem Wert gehalten. Der gebildete gelbe voluminöse Niederschlag wird über einen G4 Filtertiegel abgesaugt, je zweimal mit eisgekühltem tridestilliertem Wasser und Ethanol gewaschen und im Vakuumexsiccator über Phosphorpentoxid getrocknet.

| | | | | | |
|---|---|---|---|---|---|
| Aussehen: | gelber Feststoff | | | | |
| Schmelzpunkt: | >310 °C Zersetzung | | | | |
| Ausbeute: | 3.0 g = 7.6 mmol = 80 % | | | | |
| | | | | | |
| Elementaranalyse: | berechnet für C₇H₁₆Cl₂N₂Pt | | | 394.21 g/mol | |
| | C | H | N | Cl | Pt |
| berechnet | 21.33 | 4.09 | 7.11 | 17.99 | 49.49 |
| gefunden | 21.44 | 3.80 | 7.00 | | |

| | | |
|---|---|---|
| **IR-Spektrum** [370-7000 cm⁻¹,Csl], charakteristische Banden (in cm⁻¹): | | |
| | 3273, 3197 | ν(N-H) |
| | 2936, 2866 | ν(C-H) |
| | 1562 | δ(N-H) |

### Synthesebeispiel 8

### (SP-4-3)-Dichloro(4-ethyl-trans-cyclohexan-1,2-diamin)platin(II)

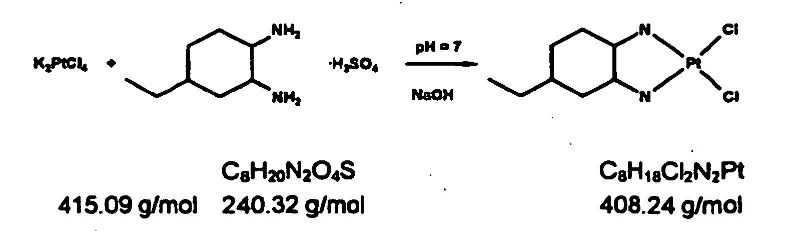

| | |
|---|---|
| Ansatz: | 2.000 g = 8.32 mmol 4-Ethyl-*trans*-cyclohexan-1,2-diamindihydrogensulfat |
| | 3.454 g = 8.32 mmol K₂PtCl₄ |

3.454 g K₂PtCl₄ werden in 50 ml tridestillierten Wasser gelöst und mit 2.000 g 4-Ethyl-*trans*-cyclohexan-1,2-diamindihydrogensulfat suspendiert. Anschließend wird der pH-Wert mit 0.5 M NaOH auf 7 eingestellt und mit einem 718 Stat Titrino der Fa. Methrom mit 0.1 M NaOH auf diesem Wert gehalten. Der gebildete gelbe voluminöse Niederschlag wird über einen G4 Filtertiegel abgesaugt, je zweimal mit eisgekühltem tridestilliertem Wasser und Ethanol gewaschen und im Vakuumexsiccator über Phosphorpentoxid getrocknet.

| | | | | | |
|---|---|---|---|---|---|
| Aussehen: | | gelber Feststoff | | | |
| Schmelzpunkt: | | >310 °C Zersetzung | | | |
| Ausbeute: | | 3.238 g = 7.93 mmol = 95 % | | | |
| | | | | | |
| Elementaranalyse: | | berechnet für C₈H₁₈Cl₂N₂Pt | | | 408.24 g/mol |
| | C | H | N | Cl | Pt |
| berechnet | 23.54 | 4.44 | 6.86 | 17.37 | 47.79 |
| gefunden | 23.52 | 4.18 | 6.63 | | |
| | | | | | |

| | | |
|---|---|---|
| **IR-Spektrum** [370-7000 cm⁻¹,Csl], charakteristische Banden (in cm⁻¹): | | |
| | 3272,3196 | ν(N-H) |
| | 2933, 2861 | ν(C-H) |
| | 1558 | δ(N-H) |

### Synthesebeispiel 9

### 4,5-Dimethyl-1-cyclohexen (2 Wege: Mesylat/Tosylat)

### 4,5-Di(hydroxymethyl)cyclohexen

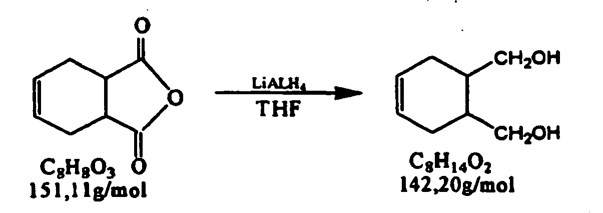

| | |
|---|---|
| Ansatz: | 30g = 0.2 mol cis-1,2,3,6-Tetrahydrophtalsäureanhydrid |
| | 45g = 1.2 mol LiAlH₄ |
| | 375 ml abs. Tetrahydrofuran |

30 g cis-1,2,3,6-Tetrahydrophtalsäureanhydrid in 150 ml abs. THF werden durch einen Tropftrichter einer Lösung von 45 g Lithiumaluminiumhydrid in 900 ml abs. THF zugegeben und 48 Stunden auf Rückfluß erhitzt. Danach wird das Gemisch zuerst auf Raumtemperatur und dann im Eis-Kochsalz-Bad auf etwa - 15°C abgekühlt und die nicht verbrauchten Anteile des Hydrids vorsichtig mit Wasser zerstört. Durch Zugabe von verdünnter Schwefelsäure werden die Salze gelöst und durch Zugabe von Diethylether wird die organische von der wäßrigen Phase getrennt.

Die organische Phase wird mit einer gesättigten Na₂CO₃-Lösung gewaschen und mit Na₂SO₄ getrocknet. Die Lösungsmittel d.h. Ether und THF werden im Vakuum abrotiert.

| | |
|---|---|
| Aussehen: | gelbe ölige Flüssigkeit |
| Ausbeute: | 27g = 0.19 mol = 95,6% |

### Synthesebeispiel 10

### 4,5-Di(methylsulphonat)cyclohexe

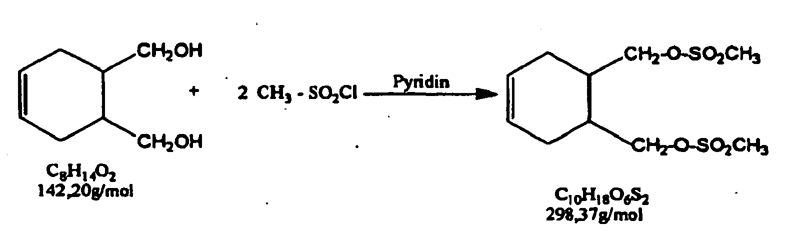

| | |
|---|---|
| Ansatz: | 28g = 0.19 mol 4,5-Di(hydroxymethyl)-1-cyclohexen |
| | 46g = 0.40 mol Methansulfochlorid ( Mr = 114,55 ) |
| | 330 ml abs. Pyridin |

46 g Methansulfochlorid werden in kleinen Portionen einer kalten Lösung von 28 g 4,5-Di(hydroxymethyl)-1-cyclohexen in 200 ml abs. Pyridin zugegeben. Das Gemisch wird 4-5 Stunden bei einer Temperatur von 3-4°C gerührt und danach weitere 24 Stunden bei 2°C gelagert. Nach Zugabe von einem Überschuss an verdünnter Salzsäure wird die Lösung mit Chloroform extrahiert. Die Chlorofom-Phase wird mit einer gesättigten Na₂CO₃-Lösung und Wasser gewaschen, mit Na₂SO₄ getrocknet und das Lösungsmittel im Vakuum abrotiert. Das Produkt wird anschliessend aus Methanol und Petroleumbenzin (40-60°C) umkristallisiert.

| | | | | |
|---|---|---|---|---|
| Aussehen: | farblose Kristalle | | | |
| m.p.: | 86-87°C | | | |
| Ausbeute: | 39 g = 0.13 mol =68.8% | | | |
| Elementaranalyse: | | berechnet für C₁₀H₁₈S₂O₆ | | |
| | C | H | O | S |
| berechnet | 40.26 | 6.08 | 32.17 | 21.49 |
| gefunden: | 40.17 | 8.08 | | 21.42 |
| | | | | |

### Synthesebeispiel 11

### 4,5-Di(methyltosylat)cyclohexen

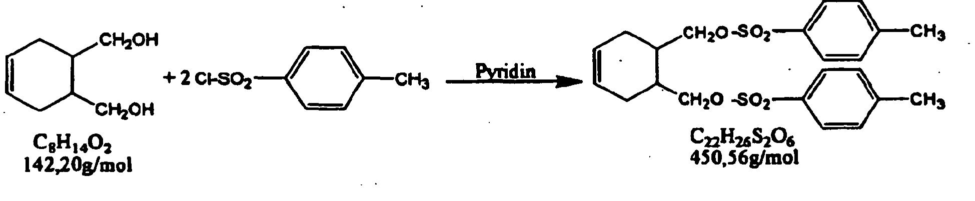

| | |
|---|---|
| Ansatz: | 30g = 0.21 mol 4,5-Di(hydroxymethyl)-1-cyclohexen |
| | 84g = 0.44 mol p-Toluol-4-sulfochlorid (Mr = 190,65) |
| | 300 ml abs. Pyridin |

84 g p-Toluol-4-sulfochlorid in 200 ml Pyridin werden in kleinen Portionen einer kalten Lösung von 30 g 4,5'-Di(hydmxymethyl)-1-cyclohexen in 60 ml abs. Pyridin zugegeben.

Das Gemisch wird 4-5 Stunden bei einer Temperatur von 3-4°C gerührt und danach weitere 24 Stunden bei 2°C gelagert. Am nächsten Tag wird das Gemisch mit 90 ml konzentrierter HCl und 180 ml Eis versetzt worauf sich ein braunes Öl bildet. Die wäßrige Phase wird zweimal mit 50 ml Ether extrahiert. Die Ether-Phasen und das Ditosylat werden einmal mit Wasser danach mit einer gesättigten NaHCO₃-Lösung und anchliessend wieder mit Wasser gewaschen. Die Etherphase wird mit Na₂SO₄ getrocknet und das Lösungsmittel im Vakuum abrotiert.

| | | | | |
|---|---|---|---|---|
| Aussehen: | farblose Kristalle | | | |
| m.p.: | 93-94°C | | | |
| Ausbeute: | 49 g = 0.11 mol = 52.4 % | | | |
| Elementaranalyse: berechnet für C₂₂H₂₆S₂O₈ | | | | |
| | C | H | O | S |
| berechnet: | 58.65 | 5.82 | 21.31 | 14.23 |
| gefunden: | 58.06 | 5.84 | | 13.91 |
| | | | | |

### Synthesebeispiel 12

### 4,5-Dimemyl-1-cyclohexen aus dem Di(methylsulphonat)

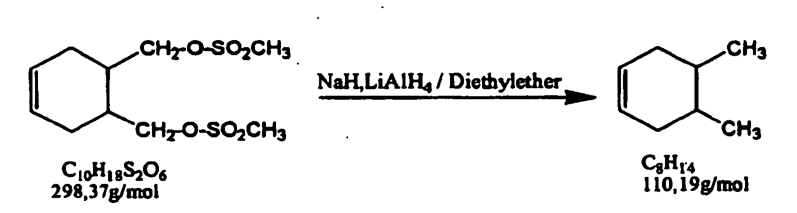

| | |
|---|---|
| Ansatz: | 19.0 g = 0.064 mol 4,5-Di(methansulfonat)cyclohexen |
| | 10.6 g = 0.28 mol LiAlH₄ |
| | 6.40 g = 0.27 mol NaH |

11.2 g LiAlH₄ und 6.7 g NaH in 300 ml abs. Ether werden 30 min. auf Rückfluß erhitzt und anschliessend auf -5°C abgekühlt. 19 g des Dimesylats werden in fester Form zugegeben und die Temperatur eine Stunde auf -5°c gehalten. Danach wird das Gemisch über Nacht auf Rückfluß erhitzt.

Die Hydrid-Überschüsse werden durch Zusatz von gesättigter Ammoniumchlorid-Lösung zerstört und das Gemisch eine Stunde gerührt. Danach wird der Feststoff abfiltriert und mit Ether gewaschen. Die organische Phase wird mit Na₂SO₄ getrocknet, und Ether wird abrotiert. Anschliessend wird das Produkt bei 123-125°C abdestilliert.
Aussehen: farblose ölige Flüssigkeit
Ausbeute: 2.3 g = 20.9 mmol = 32.6%

### Synthesebeispiel 13

### 4,5-Dimethyl-1-cyclohexen aus dem Di(methyltosylat)

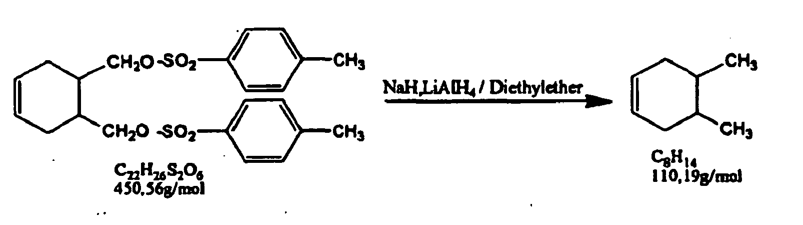

| | |
|---|---|
| Ansatz: | 48.0 g = 0.1 mol 4,5-Di(methyltosylat)cyclohexen |
| | 17.0 g = 0.44 mol LiAlH₄ |
| | 10,0 g = 0.42 mol NaH |

17 g LiAlH₄ und 10 g NaH in 500 ml abs. Ether werden 30 min. auf Rückfluß erhitzt und anschliessend auf -5°C abgekühlt. 48 g des Ditosylats werden in fester Form zugegeben und die Temperatur eine Stunde auf -5°c gehalten. Danach wird das Gemisch über Nacht auf Rückfluß erhitzt.

Die Hydrid-Überschüsse werden durch Zusatz von gesättigter Ammoniumchlorid-Lösung zerstört und das Gemisch eine Stunde gerührt. Danach wird der Feststoff abfiltriert und mit Ether gewaschen. Die organische Phase wird mit Na₂SO₄ getrocknet, und Ether wird abrotiert. Anschliessend wird das Produkt bei 123-125°C abdestilliert.
Aussehen: farblose ölige Flüssigkeit
Ausbeute: 2.3 g = 20.9 mmol = 20.9%

### Synthesebeispiel 14

### 4,5-Dimethyl-cyclohexan-1,2-diazid

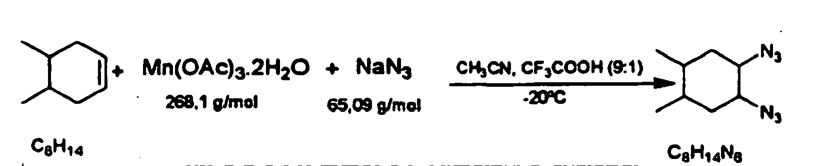

| | |
|---|---|
| Ansatz: | 1.90 g = 17.2 mmol 4,5-Dimethyl-1-cyclohexen 94.23 g/mol |
| | 13.9 g = 51.6 mmol Mn(OAc)₃.2H₂O |
| | 5.60 g = 86.0 mmol NaN₃ |

1.9 g 4,5-Dimethyl-1-cyclohexen und 20 ml TFA werden bei -20°C und unter N₂-Atmosphäre einer Suspension aus Mn(OAc)₃.2H₂O und NaN₃ in 200 ml Acetonitril zugegeben. Das Gemisch wird 90 Minuten bei -19 bis -21°C gerührt und anschließend mit einer 10%igen NaHSO₃-Lösung verdünnt. Die Lösung wird dann mit 3 x 50 ml Petroleumbenzin (Kp = 40-60°C) extrahiert.

Die organischen Phasen werden mit gesättigter Na₂CO₃-Lösung und danach mit gesättigter NaCl-Lösung gewaschen und mit Na₂SO₄ getrocknet. Das Lösungsmittel wird anschließend im Vakuum abrotiert.

| | |
|---|---|
| Ausbeute: | 2 g = 10.3 mmol = 60 % |
| Aussehen: | farblose Flüssigkeit |

### Synthesebeispiel 15

### 4,5-Dimethyl-cyclohexan-1,2-diamin

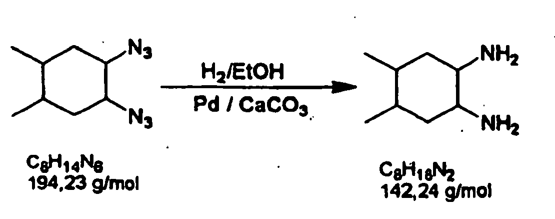

| | |
|---|---|
| Ansatz: | 1.9 g = 9.8 mmol 4,5-Dimethyl-cyclohexan-1,2-diazid |
| | 0.8 g Pd/CaCO₃ |

1.9 g 4,5-Dimethyl-cyclohexan-1,2-diazid in 60 ml abs. Ethanol werden mit 0.8 g Lindlar's Katalysator versetzt und in einem Autoklaven bei einem H₂-Druck von 3-3.5 bar während 45 Stunden zum Diamin reduziert. Nach Ende der Reduktion wird der Katalysator abfiltriert und Ethanol im Vakuum abrotiert.
Ausbeute: 1.34 g = 9.42 mmol = 96% 4,5-Dimethyl-1,2-cyclohexandiamin
Charakterisierung als Dihydrogensulfat:

4,5-Dimethyl-cyclohexan-1,2-diamin wird mit Schwefelsäure ( c = 3 mol/l) angesäuert. Der ausgefallene Feststoff wird mit Ethanol aufgenommen und auf 50°C aufgewärmt. Die Verunreinigungen lösen sich auf und das weiße 4,5-Dimethyl-1,2-diaminocyclohexan-dihyrogensulfat wird Ober einen G4-Filtertiegel abgesaugt und im Vakuum über Phosphorpentoxid getrocknet.

| | | | | | |
|---|---|---|---|---|---|
| Elementaranalyse: berechnet für C₈H₁₈N₂.H₂SO₄ | | | | | |
| | C | H | N | O | S |
| berechnet | 39.98 | 8.39 | 11.66 | 26.63 | 13.34 |
| gefunden: | 39.15 | 8.55 | 11.23 | | 13.43 |

NMR-Spektrum: 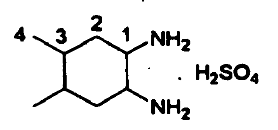 ¹H-NMR in D₂O:
δ = 0,82-0,87 [m, 3H, H(4) u. H(4'), ³J_{H,H}], 1,68 [m, 1H, H(3) u. H(3')], 1,95 [m, 2H, H(2) u. H(2')], 3,34-3,4 [m, 2H, H(1) und H(1')], 3,47-3,54 [m, 2H, H(2) und H(2')].
¹³C-NMR in D₂O:
δ = 10,94 [C(4')], 18,21 [C(4)], 31,61[C(2')], 31,67 [C(3')], 32,65 [C(3)], 36,55 [C(2)], 48,71 [C(1')], 52,82 [C(1)].

### Synthesebeispiel 16

### 4-Propyl-1-cyclohexen

### a) 4-Propyl-1-cyclohexanol

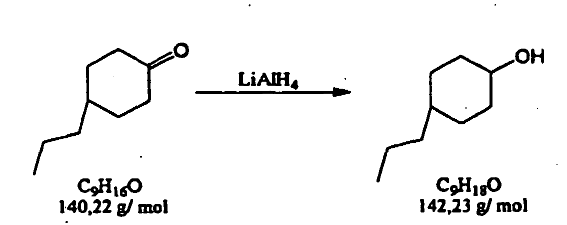

| | |
|---|---|
| Ansatz: | 20 g = 143 mmol 4-Propyl-1-cyclohexanon |
| | 10 g LiAlN₄ |

20 g 4-Propyl-1-cyclohexanon werden durch einen Tropftricher und unter Argon-Atmosphäre einer gekühlten Lösung von 10 g LiAlH₄ in 500 ml abs. THF langsam zugetropft.

Nach Ende der Zugabe wird das Gemisch zum Rückfluß erhitzt und 48 Stunden bei dieser Temperatur gerührt. Nach Ende der Reduktion werden 500 ml Diethylether zugegeben und durch tropfenweise Zugabe von dest. Wasser die nicht umgesetzten Reste vom LiAlH₄ zerstört. Danach wird das Gemisch mit 200 ml 10%ige Schwefelsäure versetzt um die anorganischen Salze zu lösen. Die Ätherphase wird dann abgetrennt und die wäßrige Phase zweimal mit 50 ml Äther extrahiert. Die kombinierten Ätherphasen werden mit Na₂SO₄ getrocknet, Äther und THF werden abrotiert.

| | |
|---|---|
| Aussehen: | farblose ölige Flüssigkeit |
| Ausbeute: | 19,3 g = 135 mmol = 94% |

### b) 4-Propyl-1-cyclohexen

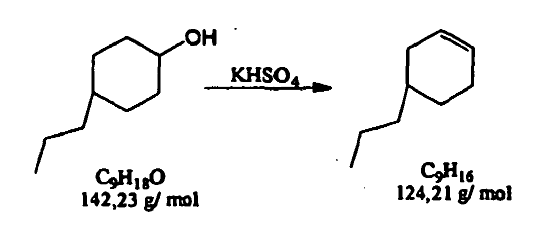

| | |
|---|---|
| Ansatz: | 19.0 g = 133 mmol 4-Propylcyclohexanol |
| | 16.0 g KHSO₄ |

19 g 4-Propyl-1-cyclohexanol werden mit 18 g KHSO₄ versetzt und eine Woche bei 70°C gerührt. Nach Zugabe von Ethylacetat wird KHSO₄ abfiltriert. Ethylacetat wird abgedampft und das Produkt durch fraktioniert Destillation erhalten.
Ausbeute: 13,7 g = 110 mmol = 83 %
Aussehen: farblose Flüssigkeit
Kp = 151-153 °C

### Synthesebeispiel 17

### 4-Proyl-cyclohexan-1,2-diazid

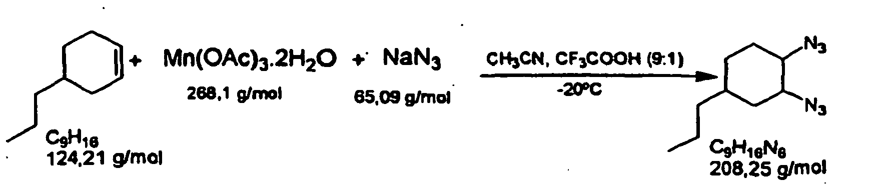

| | |
|---|---|
| Ansatz: | 4.80 g = 38.6 mmol 4-Propyl-1-cyclohexen |
| | 31.1 g = 116 mmol Mn(OAc)₃.2H₂O |
| | 12.6 g = 196 mmol NaN₃ |

4.8 g 4-Propyl-1-cyclohexen und 44 ml TFA werden bei -20°C und unter N₂-Atmosphäre einer Suspension aus Mn(OAc)₃.2H₂O und NaN₃ in 440 ml Acetonitril zugegeben. Das Gemisch wird 3 Stunden bei -19 bis -21°C gerührt und anschließend mit einer 10%igen NaHSO₃-Lösung verdünnt. Die farblose Lösung wird dann mit 3 x 50 ml Petroleumbenzin (Kp = 40-60°C) extrahiert.

Die organischen Phasen werden mit gesättigter Na₂CO₃-Lösung und danach mit gesättigter NaCl-Lösung gewaschen und mit Na₂SO₄ getrocknet. Das Lösungsmittel wird anschließend im Vakuum abrotiert.

| | |
|---|---|
| Ausbeute: | 29,4 g = 163 mmol = 78,5 % |
| Aussehen: | farblose Flüssigkeit |

### Synthesebeispiel 18

### 4-Propyl-trans-cyclohexan-1,2-diamin

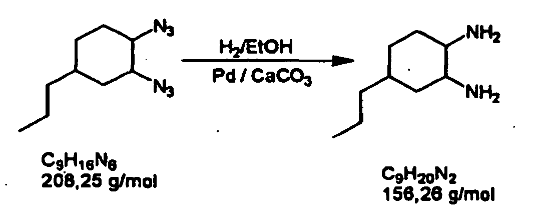

| | |
|---|---|
| Ansatz: | 4 g = 19.2 mmol 4-Propyl-cyclohexan-1,2-diazid |
| | 1.6 g Pd / CaCO₃ |

4 g 4-Ethyl-cyclohexan-1,2-diazid in 60 ml abs. Ethanol werden mit 1.6 g Lindlar's Katalysator versetzt und in einem Autoklaven bei einem H₂-Druck von 3 bar während 48 Stunden zum Diamin reduziert. Nach Ende der Reduktion wird der Katalysator abfiltriert und Ethanol im Vakuum abrotiert.

### Charakterisierung als Dihydrogensulfat:

4-Propyl-1,2-diaminocyclohexan wird mit Schwefelsäure( c = 3 mol/l) angesäuert. Der ausgefallene Feststoff wird mit Ethanol aufgenommen und auf 50°C aufgewärmt. Die Verunreinigungen lösen sich auf und das weiße 4-Propylcyclohexan-1,2-diamin-dihyrogensultat wird über einen G4-Filtertiegel abgesaugt und im Vakuum über P₂O₅ getrocknet.

### Synthesebeispiel 19

### 4-t-Butyl-1-cyclohexen

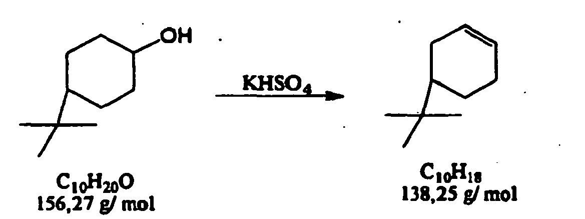

| | |
|---|---|
| Ansatz: | 20.0 g = 120 mmol 4-Propylcyclohexanol |
| | 17.0 g KHSO₄ |

20 g 4-t-Butyl-1-cyclohexanol werden mit 17 g KHSO₄ versetzt und eine Woche bei 95°C gerührt. Nach Zugabe von Ethylacetat wird KHSO₄ abfiltriert. Ethylacetat wird abgedampft und das Produkt durch fraktionierte Destillation erhalten.
Ausbeute: 11 g = 80 mmol = 65 %
Aussehen: farblose Flüssigkeit
Kp = 168-171 °C

### Synthesebeispiel 20

### 4-t-Butyl-cyclohexan-1,2-diazid

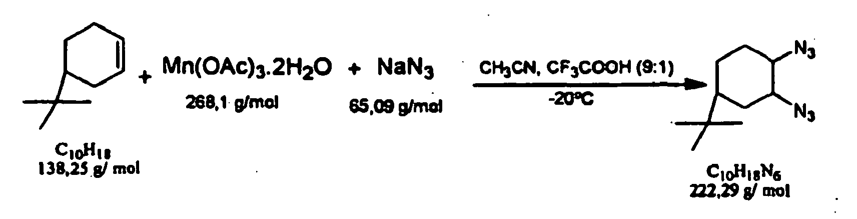

| | |
|---|---|
| Ansatz: | 5.0 g = 36 mmol 4-t-Butyl-1-cyclohexen |
| | 29 g = 108 mmol Mn(OAc)₃.2H₂O |
| | 11.8 g = 180 mmol NaN₃ |

5 g 4-t-Butyl-1-cyclohexen und 42 ml TFA werden bei -20°C und unter N₂-Atmosphäre einer Suspension aus Mn(OAc)₃.2H₂O und NaN₃ in 420 ml Acetonitril zugegeben. Das Gemisch wird 3 Stunden bei -19 bis -21°C gerührt und anschließend mit einer 10%igen NaHSO₃-Lösung verdünnt. Die farblose Lösung wird dann mit 3 x 50 ml Petroleumbenzin (Kp = 40-60°C) extrahiert. Die organischen Phasen werden mit gesättigter Na₂CO₃-Lösung und danach mit gesättigter NaCl-Lösung gewaschen und mit Na₂SO₄ getrocknet. Das Lösungsmittel wird anschließend im Vakuum abrotiert.

| | |
|---|---|
| Ausbeute: | 6.39 g = 28.7 mmol = 80 % |
| Aussehen: | farblose Flüssigkeit |

### Synthesebeispiel 21

### 4-t-Butyl-trans-cyclohexan-1,2-diamin

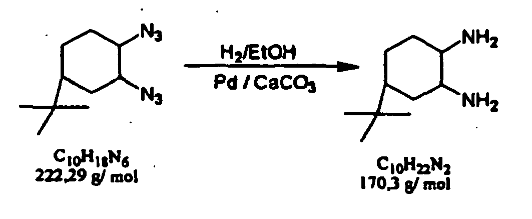

| | |
|---|---|
| Ansatz: | 4 g = 19.2 mmol 4-t-Butyl-cyclohexan-1,2-diazid |
| | 1.6g Pd/CaCO₃ |

4 g 4-Ethyl-cyclohexan-1,2-diazid in 60 ml abs. Ethanol werden mit 1.6 g Lindlar's Katalysator versetzt und in einem Autoklaven bei einem H₂-Druck von 3 bar während 48 Stunden zu Diamin reduziert. Nach Ende der Reduktion wird der Katalysator abfiltriert und Ethanol im Vakuum abrotiert.

### Charakterisierung als Dihydrogensulfat:

4-t-Butyl-1,2-diaminocyclohexan wird mit Schwefelsäure( c = 3 mol/l ) angesäuert. Der ausgefallene Feststoff wird mit Ethanol aufgenommen und auf 50°C aufgewärmt. Die Verunreinigungen lösen sich auf und das weiße 4-t-Butylcyclohexan-1,2-diamin-dihyrogensulfat wird über einen G4-Filtertiegel abgesaugt und im Vakuum über P₂O₅ getrocknet.

### Synthesebeispiel 22

### 4-Phenyl-1-cyclohexen

### a) 4-Phenyl-1-cyclohexanol

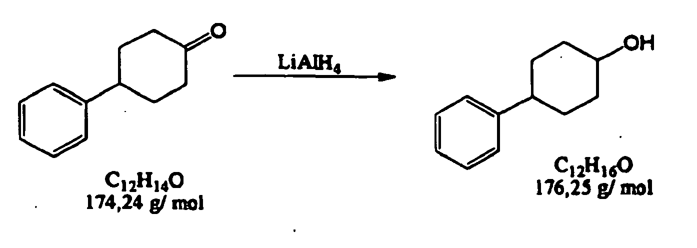

| | |
|---|---|
| Ansatz: | 20 g = 115 mmol 4-Phenyl-1-cyclohexanon |
| | 10 g LiAlH₄ |

20 g 4-Phenyl-1-cyclohexanon werden durch einen Tropftrichter und unter Argon-Atmosphäre einer gekühlten Lösung von 10 g LiAlH₄ in 500 ml abs. THF langsam zugetropft.

Nach Ende der Zugabe wird das Gemisch zum Rückfluß erhitzt und 48 Stunden bei dieser Temperatur gerührt. Nach Ende der Reduktion werden 500 ml Diethylether zugegeben und durch tropfenweise Zugabe von dest. Wasser die nicht umgesetzten Reste vom LiAlH₄ zerstört. Danach wird das Gemisch mit 200 ml 10%ige Schwefelsäure versetzt um die anorganischen Salze zu lösen. Die Ätherphase wird dann abgetrennt und die wäßrige Phase zweimal mit 50 ml Äther extrahiert. Die kombinierten Ätherphasen werden mit Na₂SO₄ getrocknet, Äther und THF werden abrotiert.
Aussehen: weißer Feststoff
Ausbeute: 18 g = 102 mmol = 89%
Schmelzpunkt: 100,5-114,1°C

### b) 4-Phenyl-1-cyclohexen

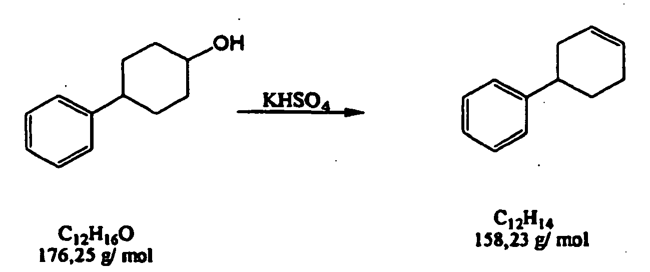

| | |
|---|---|
| Ansatz: | 18.0 g = 102 mmol 4-Phenylcyclohexanol |
| | 16.0 g KHSO₄ |

19 g 4-Propyl-1-cyclohexanol werden mit 16 g KHSO₄ versetzt und eine Woche bei 125°C gerührt. Nach Zugabe von Ethylacetat wird KHSO₄ abfiltriert. Ethylacetat wird abgedampft und das Produkt durch Vakuumdestillation erhalten.
Ausbeute: 12 g = 76 mmol = 75 %
Aussehen: farblose Flüssigkeit
Kp = 56-59 °C bei 1,1-1,3 mbar

### Synthesebeispiel 23

### 4-Phenyl-cyclohexan-1,2-diazid

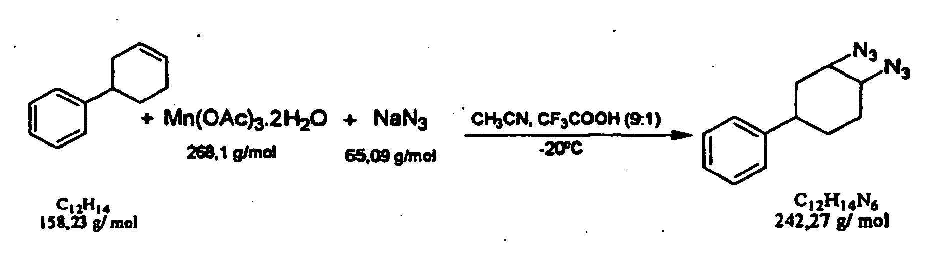

| | |
|---|---|
| Ansatz: | 5.3 g = 33.5 mmol 4-Phenyl-1-cyclohexen |
| | 27 g = 101 mmol Mn(OAc)₃.2H₂O |
| | 11 g = 168 mmol NaN₃ |

5.3 g 4-Phenyl-1-cyclohexen und 40 ml TFA werden bei -20°C und unter N₂-Atmosphäre einer Suspension aus Mn(OAc)_{3·}2H₂O und NaN₃ in 400 ml Acetonitril zugegeben. Das Gemisch wird 3 Stunden bei -19 bis -21°C gerührt und anschließend mit einer 10%igen NaHSO₃-Lösung verdünnt. Die farblose Lösung wird dann mit 3 x 50 ml Petroleumbenzin (Kp = 40-60°C) extrahiert.

Die organischen Phasen werden mit gesättigter Na₂CO₃-Lösung und danach mit gesättigter NaCl-Lösung gewaschen und mit Na₂SO₄ getrocknet. Das Lösungsmittel wird anschließend im Vakuum abrotiert.

| | |
|---|---|
| Ausbeute: | 6 g = 24.8 mmol = 74 % |
| Aussehen: | farblose Flüssigkeit |

### Synthesebeispiel 24

### 4-Phenyl-trans-cyclohexan-1,2-diamin

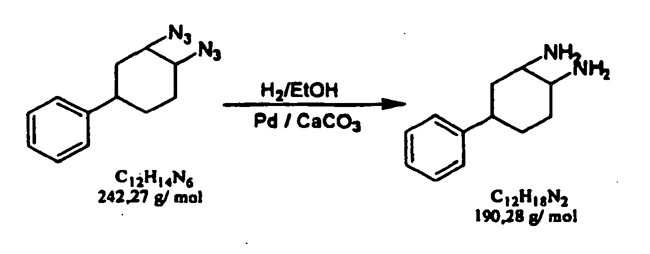

| | |
|---|---|
| Ansatz: | 4 g = 19.2 mmol 4-Phenyl-cyclohexan-1,2-diazid |
| | 1.6 g Pd/CaCO₃ |

4 g 4-Phenyl-cyclohexan-1,2-diazid in 60 ml abs. Ethanol werden mit 1.6 g Lindlar's Katalysator versetzt und in einem Autoklaven bei einem H₂-Druck von 3 bar während 48 Stunden zum Diamin reduziert. Nach Ende der Reduktion wird der Katalysator abfiltriert und Ethanol im Vakuum abrotiert.

### Charakterisierung als Dihydrogensulfat

4-Phenyl-1,2-diaminocyclohexan wird mit Schwefelsäure( c = 3 mol/l ) angesäuert. Der ausgefallene Feststoff wird mit Ethanol aufgenommen und auf 50°C aufgewärmt. Die Verunreinigungen lösen sich auf und das weiße 4-Phenylcyclohexan-1,2-diamin-dihyrogensulfat wird über einen G4-Filtertiegel abgesaugt und im Vakuum über P₂O₅ getrocknet.

### Synthesebeispiel 25

### (SP-4-3)-Dichloro(4,5-dimethyl-trans-cyclohexan-1,2-diamin)platin(II)

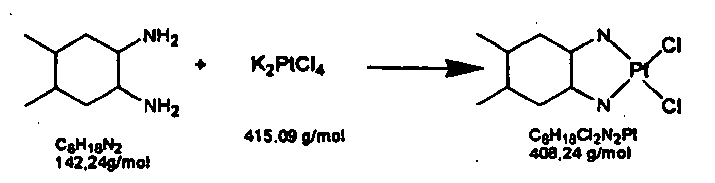

| | |
|---|---|
| Ansatz: | 0.60 g = 4.22 mmol 4,5-Dimethyl-trans-cyclohexan-1,2-diamin |
| | 1.75 g = 4.22 mmol K₂PtCl₄ |

1.75 g K₂PtCl₄ werden in 30 ml tridestilliertem Wasser gelöst und mit 0.6 g 4,5-Dimethyl-trans-cyclohexan-1,2-diamin suspendiert. Der gebildete Niederschlag wird nach und nach über einen POR 4-Glasfiltertiegel abfiltriert und im Exsiccator über Phosphorpentoxid getrocknet.

| | | | | | |
|---|---|---|---|---|---|
| Aussehen: | gelber Feststoff | | | | |
| Ausbeute: | 1.12 g = 2.74 mmol = 65% | | | | |
| | | | | | |
| Elementaranalyse: | berechnet für C₈H₁₈N₂Cl₂Pt | | | | 408.24 g/mol |
| | | | | | |
| | C | H | N | Pt | Cl |
| berechnet | 23.54 | 4.44 | 6.86 | 47.79 | 17.37 |
| gefunden | 23.32 | 4.39 | 6.59 | 47.00 | 16.81 |
| | | | | | |

### Synthesebeispiel 26

### (SP-4-3)-Dichloro(4-propyl-trans-cyclohexan-1,2-diamin)platin(II)

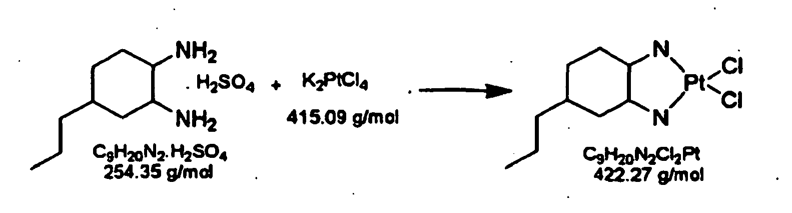

| | |
|---|---|
| Ansatz: | 1.53 g = 6.02 mmol 4-Propyl-trans-cyclohexan-1-2-diamin-dihydrogensulfat |
| | 2.50 g = 6.02 mmol K₂PtCl₄ |

2.5 g K₂PtCl₄ werden in 40 ml tridestilliertem Wasser gelöst und mit 1.53 g 4-Propyl-trans-cyclohexan-1,2-diamin-dihydrogensulfat suspendiert. Anschließend wird der pH-Wert mit 0.5 N NaOH auf 7 eingestellt und mit 0.1 N NaOH auf diesem Wert gehalten, wobei der pH-Wert kontinuierlich mit einem pH-Meter gemessen wird. Der gebildete Niederschlag wird nach und nach über einen POR 4-Glasfiltertiegel abfiltriert und im Exsiccator über Phosphorpentoxid getrocknet

| | |
|---|---|
| Aussehen: | gelber Feststoff |
| Ausbeute: | 1.83 g = 4.33mmol =72 % |

### Synthesebeispiel 27

### (SP-4-3)-Dichloro(4-6-butyl-trans-cyclohexan-1,2-diamin)platin(II)

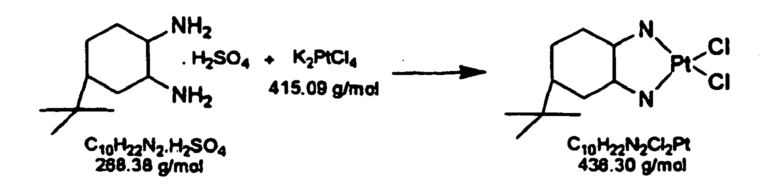

| | |
|---|---|
| Ansatz: | 1.60 g = 6.02 mmol 4-t-Butyt-trans-cyclohexan-1-2-diamin-dihydrogensulfat |
| | 2.50 g = 6.02 mmol K₂PtCl₄ |

2.5 g K₂PtCl₄ werden in 50 ml tridestilliertem Wasser gelöst und mit 1.6 g 4-t-Butyl-trans-cyclohexan-1,2-diamin-dihydrogensulfat suspendiert. Anschließend wird der pH-Wert mit 0.5 N NaOH auf 7 eingestellt und mit 0.1 N NaOH auf diesem Wert gehalten, wobei der pH-Wert kontinuierlich mit einem pH-Meter gemessen wird. Der gebildete Niederschlag wird nach und nach über einen POR 4-Glasfiltertiegel abfiltriert und im Exsiccator über Phosphorpentoxid getrocknet.

| | |
|---|---|
| Aussehen: | gelber Feststoff |
| Ausbeute: | 1.8 g = 4.12 mmol = 70 % |

### Synthesebeispiel 28

### (SP-4-3)-Dichloro(4-pheny-trans-cyclohexan-1,2-diamin)platin(II)

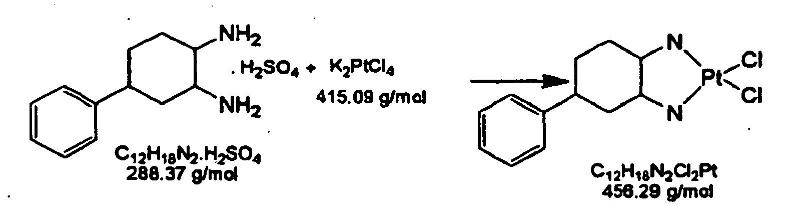

| | |
|---|---|
| Ansatz: | 1.4 g = 4.82 mmol 4-Phenyl-trans-cyclohexan-1-2-diamin-dihydrogensulfat |
| | 2.0 g= 4.82 mmol K₂PtCl₄ |

2.0 g K₂PtCl₄ werden in 40 ml tridestilliertem Wasser gelöst und mit 1.4 g 4-Phenyl-trans-cyclohexan-1,2-diamin-dihydrogensulfat suspendiert. Anschließend wird der pH-Wert mit 0.5 N NaOH auf 7 eingestellt und mit 0.1 N NaOH auf diesem Wert gehalten, wobei der pH-Wert kontinuierlich mit einem pH-Meter gemessen wird. Der gebildete Niederschlag wird nach und nach über einen POR 4-Glasfiltertiegel abfiltriert und im Exsiccator über Phosphorpentoxid getrocknet.

| | |
|---|---|
| Aussehen: | gelber Feststoff |
| Ausbeute: | 1.2 g = 2.63 mmol = 55% |

### Beispiel 1

### (SP-4-3)-(4-Methyl-trans-cyclohexan-1,2-diamin)oxalatoplatin(II)

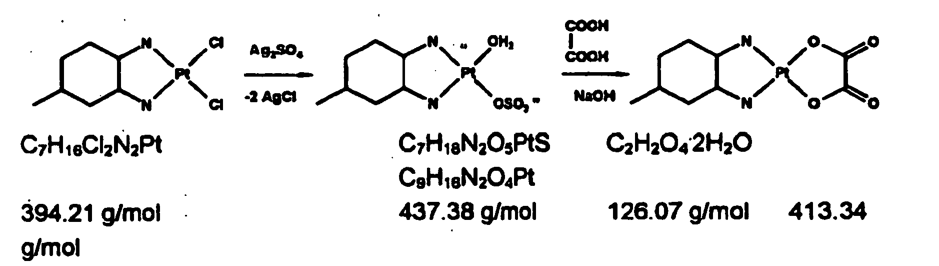

| | |
|---|---|
| Ansatz: | 0.8036 g = 2.039 mmol (4-Methyl-*trans*-dach)PtCl₂ |
| | 0.623 g = 2.00 mmol Ag₂SO₄ |
| | 0.193 g = 1.531 mmol Oxalsäuredihydrat |
| | 3 ml 0.5 M NaOH |

0.8036 g (4-Methyl-*trans*-dach)PtCl₂ werden in 30 ml tridestilliertem Wasser gut suspendiert. Dann werden 0.6433 g Ag₂SO₄ fest zugegeben und der Ansatz 2 Tage lang unter Lichtschutz gerührt.

Das ausgefallene AgCl wird abfiltriert und das Filtrat am Rotationsverdampfer zur Trockene eingedampft. Vom Rückstand werden 70 mg für die Elementaranalyse und NMR-Spektroskopie entnommen. Zu 669 mg = 1.53 mmol Aqua(4-methyl-*trans*-cyclohexan-1,2-diamin)sulfatoplatin(II) werden 0.193 g Oxalsäuredihydrat und 3 ml 0.5 M NaOH zugegeben und über Nacht gerührt. Der weiße Feststoff wird über einen G4-Glasfiltertiegel abfiltriert und im Vakuumexsiccator über Phosphorpentoxid getrocknet.

### Aqua(4-methyl-trans-cyclohexan-1,2-diamin)sulfatoplatin(II)

| | | | | | | |
|---|---|---|---|---|---|---|
| Aussehen | gelber Feststoff | | | | | |
| Ausbeute | 0.739 g = 1.690 mmol = 85% | | | | | |
| Elementaranalyse: | berechnet für C₇H₁₈N₂O₅PtS 0.2·H₂SO | | | | ₄ 437.38 g/mol | |
| | C | H | N | O | Pt | S |
| berechnet | 18.40 | 4.06 | 6.13 | 20.31 | 42.69 | 8.52 |
| gefunden | 18.54 | 3.99 | 5.94 | | | |
| gefunden | 18.57 | 4.03 | 5.95 | | | |

### (SP-4-3)-(4-Methyl-trans-cyclohexan-1,2-diamin)oxalatoplatin(II)

| | | | | | |
|---|---|---|---|---|---|
| Aussehen | weißer Feststoff | | | | |
| Schmelzpunkt | >270°C Zersetzung | | | | |
| Ausbeute | 0.310 g = 0.75 mmol = 50% | | | | |
| | | | | | |
| Elementaranalyse: | berechnet für C₉H₁₆N₂O₄Pt | | | | 411.33 g/mol |
| | C | H | N | O | Pt |
| berechnet | 26.28 | 3.92 | 6.81 | 15.48 | 47.20 |
| gefunden | 26.33 | 3.67 | 6.62 | | |
| gefunden | 26.30 | 3.70 | 6.60 | | |
| | | | | | |

**IR-Spektrum** [370-7000 cm⁻¹,Csl], charakteristische Banden (in cm⁻¹):

| | |
|---|---|
| 3243 | ν(N-H) |
| 2948, 2873 | v(C-H) |
| 1668 | νₐₛ(C=O) |
| 1663 | δ(N-H) |

**NMR-Spektren:**

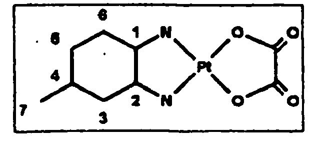

**¹H-NMR-Spektrum** in H₂O/D₂O 9/1:
δ = 0.75 - 0.87 [mm, 1H, H(5)], 0.83 [d, 3H, H(7), ³J_{H,H} = 6.60 Hz], 0.88 - 0.99 [m, 1H, H(3)], 1.19 - 1.40 [m, 2H, H(4), H(6)], 1.41 - 1.51 [m, 1H, H(5)], 1.86 - 1.97 [m, 2H, H(6) und H(3)], 2.18 - 2.41 [m, 2H, H(1) und H(2)], 3.50 - 3.60 [mm, 2H, H(1') und H(2')], 5.03 N*H*₂, 5.73 N*H*₂.

**¹³C-NMR-Spektrum** in H₂O/D₂O 911:
δ = 17.1 [C(7')], 20.4 [C(7)], 26.9 [C(6')], 27.4 [C(4')], 29.7 [C(5')], 31.0 [C(6)], 31.3 [C(4)], 32.6 [C(5)], 39.8 [C(3')], 39.9 [C(3)], 62.4 - 62.4 [4C, C(1), C(2), C(1') und C(2')], 168.7 [2C, C=O].

**¹⁵N-Signal aus dem ¹⁵N,¹H-COSY-Spektrum** in H₂O/D₂O 9/1:
δ = -34.5815.73 [*N*H₂).

### Beispiel 2

### (SP-4-3)-(4-Ethyl-trans-cyclohexan-1,2-diamin)oxalatoplatin(II)

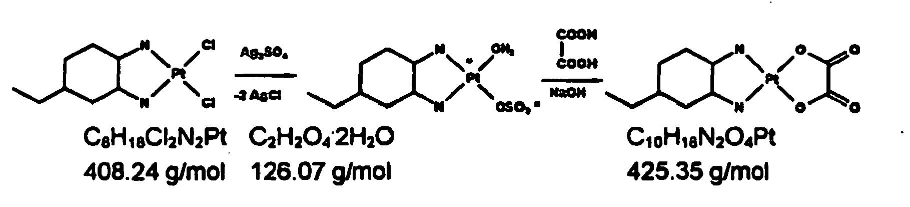

| | |
|---|---|
| Ansatz: | 0.8052 g = 1.972 mmol (4-Ethyl-*trans*-dach)PtCl₂ |
| | 0.584 g = 1.87 mmol Ag₂SO₄ |
| | 0.248 g = 1.972 mmol Oxalsäuredihydrat |
| | 4 ml 0.5 M NaOH |

0.8052 g (4-Ethyl-*trans*-dach)PtCl₂ werden in 30 ml tridestilliertem Wasser gut suspendiert. Dann werden 0.584g Ag₂SO₄ fest zugegeben und der Ansatz 5 Tage lang unter Lichtschutz gerührt. Das ausgefallene AgCl wird abfiltriert und das Filtrat am Rotationsverdampfer zur Trockene eingedampft. Zum Rückstand werden 0.248 g Oxalsäuredihydrat und 4 ml 0.5 M NaOH zugegeben und über Nacht gerührt. Der weiße Feststoff wird über einen G4-Glasfiltertiegel abfiltriert und im Vakuumexsiccator über Phosphorpentoxid getrocknet.

| | | | | | |
|---|---|---|---|---|---|
| Aussehen | | weißer Feststoff | | | |
| Schmelzpunkt | | >290°C Zersetzung | | | |
| Ausbeute | | 0.170 g = 1.48 mmol = 72% | | | |
| | | | | | |
| Elementaranalyse: | | berechnet für C₁₀H₁₈N₂O₄Pt | | | 425.35 g/mol |
| | C | H | N | O | Pt |
| berechnet | 28.24 | 4.27 | 6.59 | 15.05 | 45.87 |
| gefunden | 28.00 | 4.02 | 6.75 | | |
| | | | | | |

**IR-Spektrum** [370-7000 cm⁻¹,Csl], charakteristische Banden (in cm⁻¹):

| | |
|---|---|
| 3100 | v(N-H) |
| 2960, 2934, 2861 | v(C-H) |
| 1706 | νₐₛ(C=O) |
| 1665 | δ(N-H) |

**NMR-Spektren:**

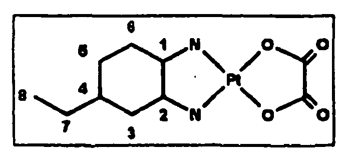

**¹H-NMR-Spektrum** in H₂O/D₂O 9/1:
δ = 0.69 - 0.85 [m, 1 H, H(5)], 0.75 [t 3H, H(8), ³J_{H,H} = 7.09 Hz], 0.86 - 0.99 [m, 1H, H(3)], 1.07 - 1.31 [m, 4H, H(4), H(6), H(7)], 1.49 -1.60 [m, 1H, H(5)], 1.89 - 2.03 [m, 2H, H(6) und H(3)], 2.21 - 2.41 [m, 2H, H(1) und H(2)], 5.75 N*H*₂.

**¹³C-Signale aus dem ¹³C,¹H-COSY-NMR-Spektrum** in H₂O/D₂O 9/1:
δ = 11.1 [C(8)], 27.9 [C(7)], 30.1 [C(5)], 30.8 [C(6)], 37.5 [C(3)], 37.9 [C(4)], 62.5 [2C, C(1), C(2)].

### Beispiel 3

### (SP-4-3)-(4,5-Dimethyktrans-cyclohexan-1,2-diamin)oxalatoplatin(II)

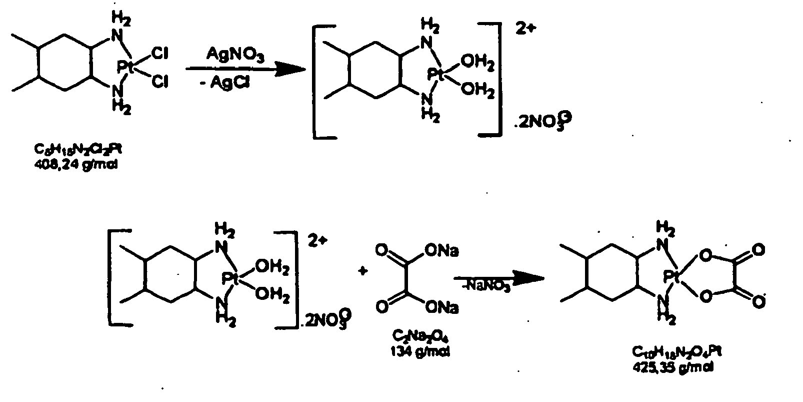

| | |
|---|---|
| Ansatz: | 600 mg = 1.47 mmol Dichloro(4,5-dimethyl-cyclohexan-1,2-diamin)platin(II) |
| | 480 mg = 2.82 mmol AgNO₃ |
| | 127 mg = 1.41 mmol Oxalsäure |
| | 2.80 ml = 2.80 mmol 1 N NaOH |

600 mg Dichloto(4,5-dimethyl-cyclohexan-1,2-diamin)platin(II) werden in 70 ml tridestilliertem Wasser suspendiert, mit 480 mg Silbernitrat versetzt und einen Tag bei Raumtemperatur gerührt. Das Ausgefallene Silberchlorid wird über einen POR 4-Glasfiltertiegel abfiltriert.

127 mg Oxalsäure werden in 2.8 ml NaOH gelöst und mit der obigen, auf die Hälfte eingeengten Lösung versetzt. Das Gemisch wird 4 Stunden bei Raumtemperatur gerührt. Das entstandene Produkt wird über einen POR 4-Glasfiltertiegel abgesaugt und im Vakuum über Phosphorpentoxid getrocknet.

| | | | | | | |
|---|---|---|---|---|---|---|
| Aussehen: | weißer Feststoff | | | | | |
| Ausbeute: | 290 mg = 0.68 mmol = 48% | | | | | |
| | | | | | | |
| Elementaranalyse: | | berechnet für C₁₀H₁₈N₂O₄Pt | | | | 425.35 g/mol |
| | | C | H | N | O | Pt |
| Berechnet: | | 28.24 | 4.27 | 6.59 | 15.05 | 45.87 |
| Gefunden: | | 28.11 | 4.02 | 6.41 | | |
| | | | | | | |

NMR-Spektrum:

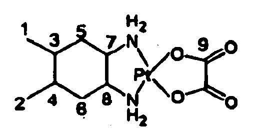

¹H-NMR in D₂O :
δ = 0,71-0,73 [d, 3H, H(1)],0,8 -0,82[d, 3H, H(2)], 1,43-1,49 [m, 1H, H(3) u. H(4)], 1,65 -1,70[m, 2H, H(5) od. H(6)], 1,79-1,84 [m, 2H, H(5) od. H(6)], 2,28-2,36 [m, 1H, H(7) od. H(8)], 2,45-2,52[m, 1 H, H(8) od. H(9)].

¹³C-NMR in D₂O :
δ = 11,44 [(C1)], 17,94 [C(2)], 32,61 [(C3)], 33,85 [C(4)], 33,89 [(C5)], 38,25 [(C6)], 58,15 [C(7) u. C(8)], 62,81 [C(7) u. C(8)], 168,71 [C(9)].

### Beispiel 4

### (SP-4-3)-(4-Propyl-trans-cyclohexan-1,2-diamin)oxalatoplatin(II)

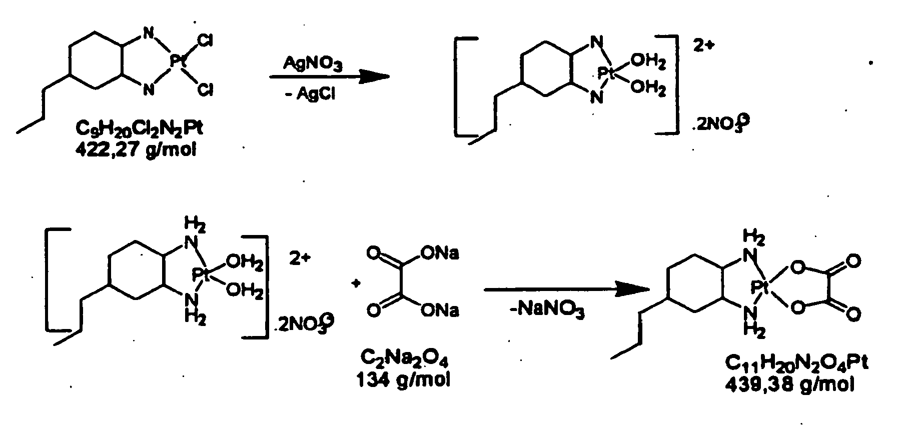

Die Synthese erfolgt analog zu Beispiel 3 aus dem Dichlorokomplex.

| | | | | |
|---|---|---|---|---|
| Elementaranalyse: | berechnet für C₁₁H₂₀N₂O₄Pt | | | 439.38 g/mol |
| | C | H | N | |
| Berechnet: | 30.07 | 4.59 | 6.37 | |
| Gefunden: | 29.78 | 4.67 | 6.45 | |

### Beispiel 5:

### (SP-4-3)-(4-t-Butyl-trans-cyclohexan-1,2-diamin)oxalatoplatin(II)

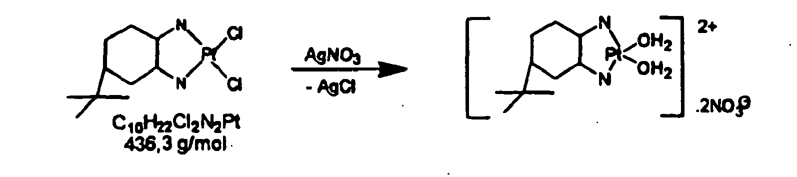

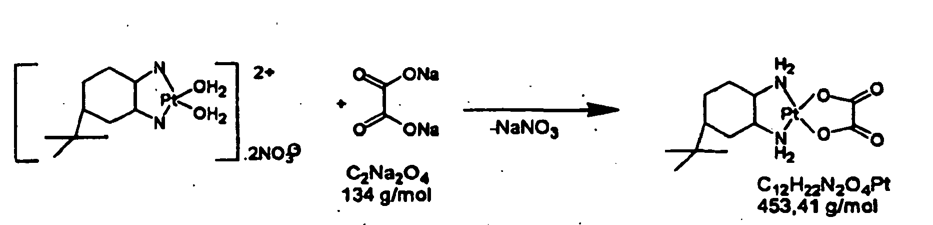

Die Synthese erfolgt analog zu Beispiel 3 aus dem Dichlorokomplex.

| | | | | |
|---|---|---|---|---|
| Elementaranalyse: | berechnet für C₁₂H₂₂N₂O₄Pt | | | 453.41 g/mol |
| | C | H | N | |
| Berechnet | 31.79 | 4.89 | 6.18 | |
| Gefunden: | 31.61 | 4.72 | 6.12 | |

### Beispiel 6:

### (SP-4-3)-(4-Phenyl-trans-cyclohexan-1,2-diamin)oxalatoplatin(II)

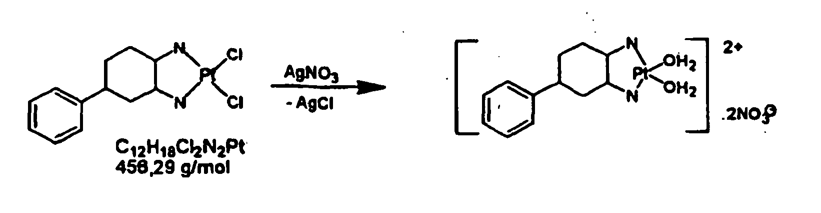

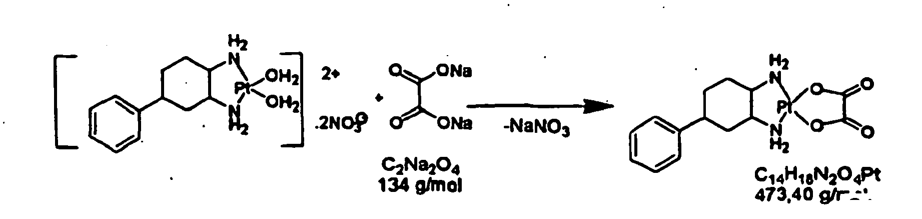

Die Synthese erfolgt analog zu Beispiel 3 aus dem Dichlorokomplex.

| | | | | |
|---|---|---|---|---|
| Elementaranalyse: | berechnet für C₁₄H₁₈N₂O₄Pt | | | 473.40 g/mol |
| | C | H | N | |
| Berechnet | 35.52 | 3.83 | 5.92 | |
| Gefunden: | 35.28 | 3.71 | 5.82 | |

### Durchführung der Racematspaltung:

Die Trennung der Enantiomere der Diamine der Synthesebeispiele 3, 6, 15, 18, 21 und 24 kann z. B. durch Salzbildung mit Weinsäure durchgeführt werden. Dazu wird entweder (R,R)- oder (S,S)-Weinsäure (je nachdem, welches Enantiomer des Diamins bevorzugt isoliert werden soll) in Wasser gelöst und bei einer Temperatur von < 70°C wird das Diamin dazugegeben. Nach der vollständigen Zugabe des Diamins wird Eisessig zugegeben, und anschließend wird die Mischung auf 5 °C abgekühlt. Das ausgefallene Salz wird abfiltriert und getrocknet. Durch Zugabe von Natronlauge und anschließende Extraktion mit einem organischen Lösungsmittel kann das freigesetzt und gewonnen werden.

Alternativ kann auch anstelle von Weinsäure die O,O-Dibenzoylweinsäure verwendet werden und man arbeitet dann in einem Wasser/Aceton-Gemisch.

Die erhaltenen enantiomerenreinen Diamine werden danach analog den Synthesebeispielen 7, 8, 25, 26, 27 und 28 zu den Dichloroplatinkomplexen und diese dann nach Aktivierung mit Silbernitrat oder Silbersulfat analog zu den Beispielen 1-6 mit Oxalsäure oder Natriumoxalat zu den Oxalatokomplexen umgesetzt.

### Zytotoxizitätstests

Die Zytotoxizität der Verbindungen nach Beispiel 1 und 2 wurde unter Verwendung der nachstehenden Zelllinien 41 M, CH1 und SW480 getestet. Dabei wurde folgende Vorgehensweise gewählt:

Die proliferationshemmende Aktivität von Oxaliplatin und dessen Derivaten [(*trans*-S,S-chxn)Pt(ox)], [(4-Methyl-*trans*-chxn)Pt(ox)] und [(4-Ethyl-*trans-*chxn)Pt(ox)]) wurde vergleichend an 3 humanen Tumorzelllinien im Mikrokultur-Tetrazollum-Test (MTT-Assay) bei kontinuierlicher Wirkstoffexposition (96 h) untersucht. Hierzu wurden adhärente Monolayer-Kulturen folgender Zelllinien: 41 M (Adenokarzinom des Ovars), CH1 (Adenokarzinom des Ovars), SW480 (Adenokarzinom des Kolons; primäre Resistenz gegen Cisplatin) verwendet.

**Kulturbedingungen:** die Zellen wurden in 75 cm²-Kulturfaschen bei 37 °C und feuchter Atmosphäre (5 % CO₂) gehalten. Als Kulturmedium wurde Minimal Essential Medium (MEM) mit 2 mmol/l I-Glutamin, 1 mmol/l Natriumpyruvat, 50 IU/ml Penizillin, 50 µg/ml Streptomyzin und 10 % hitzeinaktiviertem fetalem Rinderserum verwendet.

**Testdurchführung:** Zellsuspensionen wurden aus subkonfluenten Kulturen durch Behandeln mit Trypsin gewonnen und in definierter Dichte in 96-well-Mikrokulturplatten angesät. Die Ausgangsdichte wurde so gewählt, dass für die gesamte Testdauer ein exponentielles Wachstum der Kulturen gewährleistet war. Diese betrug für die Zelllinie 41 M 4·10⁴ Zeilen/ml, für CH1 1.25 10⁴ Zellen/ml und für SW480 1.5·10⁴ Zellen/ml. Die Platten wurden 24 h bei Standardbedingungen inkubiert, um ein Festsetzen der Zellen zu erreichen. Anschließend wurden die zu testenden Verbindungen in zehnstufigen Konzentrationsreihen im Kulturmedium (ohne Zugabe von organischen Lösungsmitteln oder Lösevermittlern) in die Platten eingebracht und für die gesamte Testdauer (96 h) belassen. Pro Experiment wurde jede Konzentration an acht Mikrokulturen der selben Zelllinie getestet. Am Ende des Experiments wurde jeweils die Anzahl der lebenden Zellen in den Mikrokulturen mittels der MTT-Farbstoffreaktion im Verhältnis zu unbehandelten Kontrollkulturen spektralphotometrisch (bei 550 nm) bestimmt. Gleichermaßen wurde zu Beginn der Wirkstoffexposition die Anzahl der lebenden Zellen in parallel angesetzten Mikrokulturplatten quantifiziert. Die Testung von [(*trans*-S,S-chxn)Pt(ox)] und dem Racemat wurde einmal, alle übrigen Experimente wurden je dreimal durchgeführt. Für die Auswertung wurden die Mittelwerte herangezogen.

### Ergebnisse mit der Zelllinie 41M

| Verbindung | IC₅₀ | GI₅₀ | TGI | LC₅₀ |
|---|---|---|---|---|
| Oxaliplatin | 4.3 ± 1.4 | 0.67 ± 0.04 | 5.5 ± 2.8 | 17.7 ± 0.7 |
| [(trans-S,S-chxn)Pt(ox)] | 3.2 | 1.11 | 4.4 | 19.8 |
| [(trans-chxn)Pt(ox)] | 4.2 | 0.99 | 5.0 | 33.1 |
| [(4-Methyl-trans-chxn)Pt(ox)] | 2.4 ± 0.5 | 0.91 ± 0.16 | 4.2 ± 2.6 | 17.9 ± 7.0 |
| [(4-Ethyl-trans-chxn)Pt(ox)] | 2.4 ± 1.4 | 0.70 ± 0.32 | 3.0 ± 0.4 | 15.1 ± 5.1 |

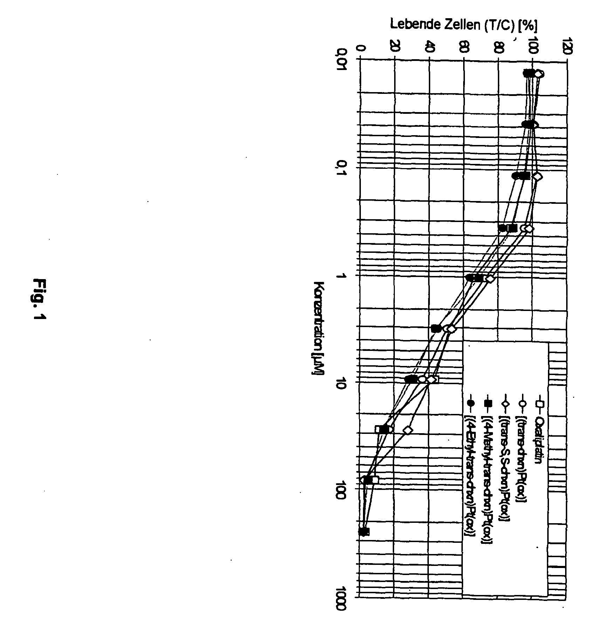

Fig. 1 zeigt eine schematische Darstellung der Ergebnisse in Abhängigkeit von der Konzentration und den lebenden Zellen (T/C) [%].

### Ergebnisse mit der Zelllinie CH1

| Substanz | IC50 | GI50 | TGI | LC50 |
|---|---|---|---|---|
| Oxaliplatin | 0.27 ± 0.22 | 0.42 ± 0.21 | 1.8 ± 0.4 | 3.6 ± 0.7 |
| [(trans-S,S-chxn)Pt(ox)] | 1.5 | 1.14 | 4.2 | 8.4 |
| [(trans-chxn)Pt(ox)] | 0.7 | 0.54 | 2.0 | 4.0 |
| [(4-Methyl-trans-chxn)Pt(ox)] | 0.34 ± 0.10 | 0.22 ± 0.03 | 0.93 ± 0.22 | 1.9 ± 0.4 |
| [(4-Ethyl-trans-chxn)Pt(ox)] | 0.30 ± 0.20 | 0.21 ± 0.14 | 0.98 ± 0.59 | 2.1 ± 1.1 |

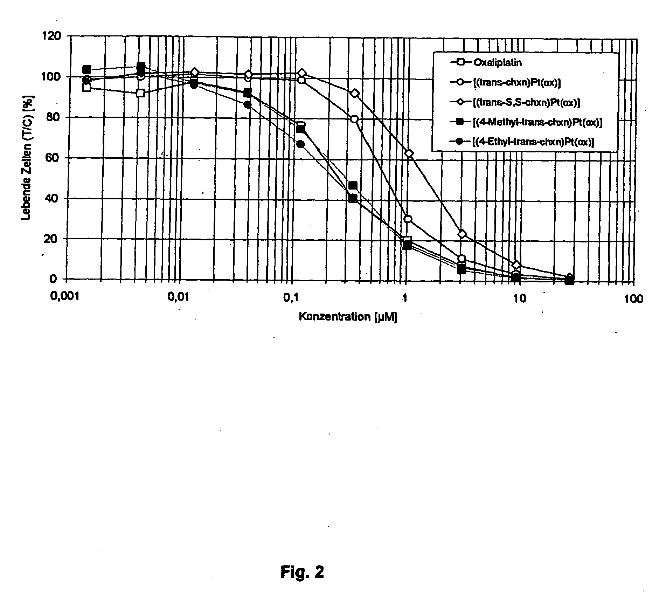

Fig. 2 zeigt eine schematische Darstellung der Ergebnisse in Abhängigkeit von der Konzentration und den lebenden Zellen (T/C) [%].

### Ergebnisse mit der Zelllinie SW480

| Substanz | IC50 | GI50 | TGI | LC50 |
|---|---|---|---|---|
| Oxaliplatin | 0.67 ± 0.28 | 0.17 ± 0.07 | 16.2 ± 2.7 | 230 ± 23 |
| [(trans-S,S- | 4.2 | 2.2 | 47.8 | 70.4 |
| chxn)Pt(ox)] | | | | |
| [(trans-chxn)Pt(ox)] | 1.6 | 0.8 | 21.8 | 104.6 |
| [(4-Methyl-trans-chxn)Pt(ox)] | 0.65 ± 0.07 | 0.31 ± 0.11 | 9.0 ± 2.9 | 73.3 ± 48.9 |
| [(4-Ethyl-trans-chxn)Pt(ox)] | 0.61 ± 0.42 | 0.30 ± 0.23 | 9.1 ± 5.3 | 21.0 ± 5.0 |

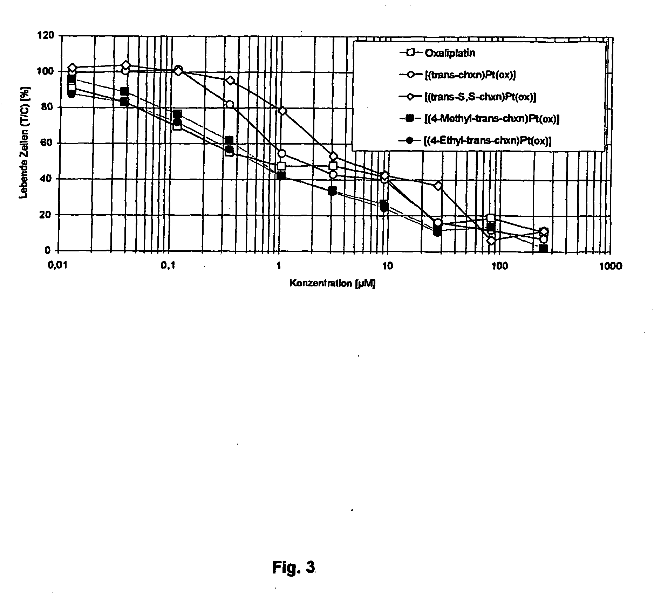

Fig. 3 zeigt eine schematische Darstellung der Ergebnisse in Abhängigkeit von der Konzentration und den lebenden Zellen (T/C) [%].

Wie den vorstehenden Ergebnissen zu entnehmen ist, im Vergleich zu Oxaliplatin, die Zytotoxizität die isomeren Gemische der Verbindungen nach Beispiel 1 und 2 vergleichbar oder besser. Das ist insbesondere überraschend, da isomere Gemische in der Regel eine geringere Zytotoxizität haben, als reine trans-R,R-Cyclohexan-1,2-diamin-platinum Verbindungen.

### In-vivo-Versuche

Durchgeführte Efficacystudien zeigen eine deutlich geringere Toxizität der neuen Derivate gegenüber Oxaliplatin selbst. Verwendung von Oxaliplatin in der höchsten Dosierung führt bis zum Tag 14 zum Sterben aller Tiere der Versuchsgruppe, wohingegen in den Gruppen, die die erfindungsgemäßen Verbindungen (4-Methyl-trans-chxn)Pt(ox) und (4-Ethyl-trans-chxn)Pt(ox) verabreicht bekommen haben, auch in der höchsten Dosierungsstufe 4/6 Tiere ((4-Methyl-trans-chxn)Pt(ox)) bzw. 6/6 Tiere ((4-Ethyl-trans-chxn)Pt(ox)) am Tag 14 noch leben.

### Schedule: Qd × 5

Weibliche, tumortragende B6D2F1-Maus, Tag 14 ab Tumortransplantation

| Substanz | Dosis mg/kg | Überlebende/ Gesamtzahl |
|---|---|---|
| (4-Methyl-trans-chxn)Pt(ox) | 1.30 | 6/6 |
| | 2.60 | 5/6 |
| | 5.20 | 4/6 |
| (4-Ethyl-trans-chxn)Pt(ox) | 1.34 | 5/6 |
| | 2.68 | 6/6 |
| | 5.35 | 6/6 |
| Oxaliplatin | 1.25 | 5/6 |
| | 2.50 | 6/6 |
| | 5.00 | 0/6 |

Die Wirksamkeit der erfindungsgemäßen Verbindungen im i. m. transplantierten murinen Lewis Lungenkarzinom war trotz der signifikant besseren Verträglichkeit vergleichbar der Wirkung von Oxaliplatin.

### L1210 Leukämie In der Maus

In diesem Experiment zeigte die neue Verbindung (4-Methyl-trans-chxn)Pt(ox) in der Mäuseleukämie sowohl eine bessere Verträglichkeit bei hoher Dosis (24 mg/kg) als auch eine bessere Wirksamkeit im Bereich von 3 mg/kg und 6 mg/kg (alle Tiere überlebten das Experiment bis zum Ende), wohingegen in der mit I-OHP behandelten Gruppe schon Todesfälle auftraten.

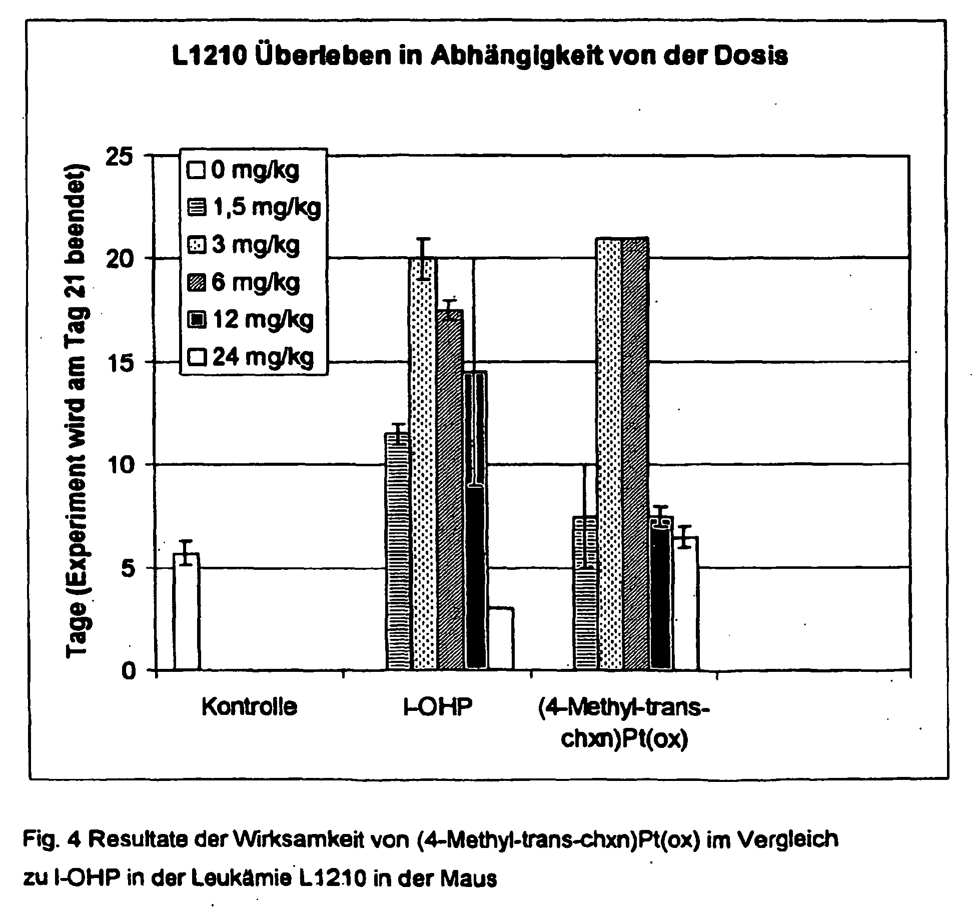

### MTD-Studie:

In dieser Studie am tumorfreien Tier zeigen die beiden neuen Derivate (4-Methyl-trans-chxn)Pt(ox) und (4-Ethyl-trans-chxn)Pt(ox) eine deutlich bessere Verträglichkeit als sie für Oxaliplatin gefunden werden kann (MTD in beiden Fällen 10 mg/kg Qd × 5, Gewichtsverlust 5 % am Tag 5, zum Vergleich: Oxaliplatin MTD = 5 mg/kg bei gleichem Schedule).

Somit ist eine bessere Verträglichkeit der neuen erfindungsgemäßen Derivate gegenüber dem etablierten Oxaliplatin im Tierversuch nachweisbar.

## Patentansprüche

1. Verbindungen der allgemeinen Formel (I), 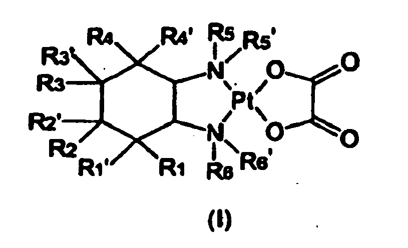 wobei die Substituenten R¹, R^{1'}, R², R^{2'}, R³, R^{3'}, R⁴, und R^{4'} unabhängig voneinander ausgewählt sind aus der Gruppe, bestehend aus Wasserstoff, verzweigten oder unverzweigten Alkyl-, verzweigten oder unverzweigten Alkenyl-, Cycloalkyl-, Cycloalkenyl-, Alyl- und Alkylarylresten,
die Substituenten R⁵, R^{5'}, R⁶, und R^{6'} unabhängig voneinander ausgewählt sind aus der Gruppe, bestehend aus Wasserstoff, verzweigten oder unverzweigten Alkyl-, und verzweigten oder unverzweigten Alkenylresten, und
wobei gegebenenfalls jeweils wenigstens zwei der Substituenten R¹, R^{1'}, R², R^{2'}, R³, R^{3'}, R⁴, und R^{4'} miteinander wenigstens einen Alkylen-, Alkenylenrest oder einen aromatischen Ring bilden können, und
wobei gegebenenfalls wenigstens eines der die Substituenten R¹, R^{1'}, R², R^{2'}, R³, R^{3'}, R⁴, und R^{4'} tragenden Kohlenstoffatome des Cyclohexanrings durch ein Heteroatom ersetzt ist, und
falls das Heteroatom Sauerstoff ist, die Substituenten R¹, R^{1'}, R², R^{2'}, R³, R^{3'}, R⁴ und/oder R^{4'} zusätzlich Hydroxyreste sein können, und
pharmazeutisch verträglich Salze davon,
mit der Maßgabe, dass
mindestens ein Substituent R¹, R^{1'}, R², R^{2'}, R³, R^{3'}, R⁴, R^{4'} ungleich Wasserstoff ist und die Substituenten R¹ oder R^{1'} und R⁴ oder R^{4'} miteinander keinen unsubstituierten C₁₋₂-Alkylenrest bilden.

2. Verbindungen nach Anspruch 1, wobei die Substituenten R¹, R^{1'}, R², R^{2'}, R³, R^{3'}, R⁴ und R^{4'} unabhängig voneinander ausgewählt sind aus der Gruppe, bestehend aus Wasserstoff, C₁-C₁₅-Alkyl-, C₂-C₁₅-Alkenyl-, C₃-C₁₅-Cycloalkyl-, C₃-C₁₅-Cycloalkenyl-, C₆-C₁₄-Aryl- und C₁-C₁₅-Alkyl-C₆-C₁₄-Arylresten.

3. Verbindungen nach einem der vorstehenden Ansprüche, wobei die Substituenten R¹, R^{1'}, R², R^{2'}, R³, R^{3'}, R⁴ und R^{4'} ausgewählt sind aus der Gruppe, bestehend aus Wasserstoff, C₁₋₁₀-Akyl-, C₃₋₁₀-Cycloalkyl- und C₆-C₁₄-Arylresten.

4. Verbindungen nach einem der vorstehenden Ansprüche, wobei die Substituenten R¹, R^{1'}, R³, R^{3'}, R⁴ und R^{4'} gleich Wasserstoff sind und einer der Substituenten R² oder R^{2'} ein C₁-C₆-Alkylrest oder ein C₆-C₁₄-Arylrest und der andere ein Wasserstoffatom ist.

5. Verbindungen nach Anspruch 4, wobei die Substituenten R² oder R^{2'} Methyl-, Ethyl-. Propyl, t-Butyl oder Phenylreste sind.

6. Verbindungen nach einem der vorstehenden Ansprüche, wobei die Substituenten R⁵, R^{5'}, R⁶ und R^{6'} Wasserstoff sind und gegebenenfalls wenigstens eines der die Substituenten R¹, R^{1'}, R², R^{2'}, R³, R^{3'}, R⁴, und R^{4'} tragenden Kohienstoflfatome des Cyclohexanrings durch ein Heteroatom ersetzt ist, und
falls das Heteroatom Sauerstoff ist, die Substituenten R¹, R^{1'}, R², R^{2'}, R³, R^{3'}, R⁴, und R^{4'} unabhängig voneinander ausgewählt sind aus der Gruppe, bestehend aus Wasserstoff, Alkyl-, Alkenyl-, Cycloalkyl-, Cycloalkenyl-, Aryl- und Alkylarylresten

7. Verbindungen gemäß Anspruch 1, worin ein oder zwei der Substituenten R¹, R^{1'}, R², R^{2'}, R³, R^{3'}, R⁴ und R^{4'} ausgewählt aus der Gruppe bestehend aus Alkyl-, Alkenyl-, Cycloalkenyl-, Aryl- und Alkylarylresten sind und die übrigen Reste Wasserstoffatome sind.

8. Verbindungen gemäß Anspruch 7, wobei die Alkylreste Methylreste sind.

9. Verbindungen gemäß einem der vorherigen Ansprüche, ausgewählt aus der Gruppe bestehend aus
(SP-4-3-)-(4-Methyl-trans-cyclohexan-1,2-diamin)oxalatoplatin(II) und
(SP-4-3-)-(4,5-Dimethyl-trans-cyclohexan-1,2-diamin)oxalatoplatin(II).

10. Verbindung nach einem der vorstehenden Ansprüche als therapeutisches oder prophytaktisches Mittel.

11. Verwendung einer Verbindung nach einem der Ansprüche 1 bis 9, zur Herstellung eines Medikaments zur Bekämpfung und/oder Prophylaxe von Tumorerkrankungen.

12. Armeimittel, umfassend eine Verbindung nach einem der Ansprüche 1 bis 9.

## Claims

1. Compounds of the general formula (I), 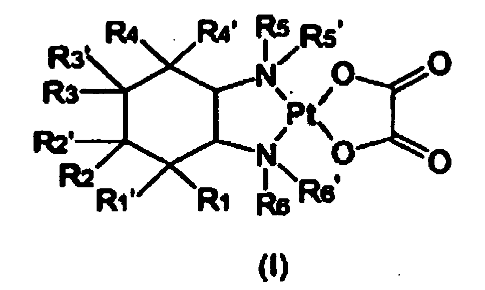 whereby the substituents R¹, R^{1'}, R², R^{2'}, R³, R^{3'}, R⁴, and R^{4'} are independently of one another selected from the group consisting of hydrogen, branched or unbranched alkyl, branched or unbranched alkenyl, cycloalkyl, cycloalkenyl, aryl and alkylaryl radicals,
the substituents R⁵, R^{5'}, R⁶, and R^{6'} are independently of one another selected from the group consisting of hydrogen, branched or unbranched alkyl and branched or unbranched alkenyl radicals, and
whereby optionally in each case at least two of the substituents R¹, R^{1'}, R², R^{2'}, R³, R^{3'}, R⁴, and R^{4'} can form with one another at least one alkylene, alkenylene radical or an aromatic ring, and
whereby optionally at least one of the carbon atoms of the cyclohexane ring bearing the substituents R¹, R^{1'}, R², R^{2'}, R³, R^{3'}, R⁴, and R^{4'} is replaced by a heteroatom, and
if the heteroatom is oxygen, the substituents R¹, R^{1'}, R², R^{2'}, R³, R^{3'}, R⁴, and/or R^{4'} can additionally be hydroxy radicals, and
pharmaceutically compatible salts of them,
provided that
at least one substituent R¹, R^{1'}, R², R^{2'}, R³, R^{3'}, R⁴, R^{4'} is not equal to hydrogen and the substituents R¹ or R^{1'} and R⁴ or R^{4'} do not form an unsubstituted C₁₋₂-alkylene radical with one another.

2. Compounds according to claim 1, whereby the substituents R¹, R^{1'}, R², R^{2'}, R³, R^{3'}, R⁴, and R^{4'} are independently of one another selected from the group consisting of hydrogen, C₁-C₁₅-alkyl, C₂-C₁₅-alkenyl, C₃-C₁₅-cycloalkyl, C₃-C₁₅-cycloalkenyl, C₆-C₁₄-aryl and C₁-C₁₅-alkyl-C₆-C₁₄-aryl radicals.

3. Compounds according to one of the previous claims, whereby the substituents R¹, R^{1'}, R², R^{2'}, R³, R^{3'}, R⁴, and R^{4'} are selected from the group consisting of hydrogen, C₁₋₁₀-alkyl, C₃₋₁₀-cycloalkyl and C₆-C₁₄-aryl radicals.

4. Compounds according to one of the previous claims, whereby the substituents R¹, R^{1'}, R³, R^{3'}, R⁴, and R^{4'} are equal to hydrogen and one of the substituents R² or R^{2'} is a C₁-C₆-alkyl radical or a C₆-C₁₄-aryl radical and the other is a hydrogen atom.

5. Compounds according to claim 4, whereby the substituents R² or R^{2'} are methyl, ethyl, propyl, t-butyl or phenyl radicals.

6. Compounds according to one of the previous claims, whereby the substituents R⁵, R^{5'}, R⁶, and R^{6'} are hydrogen and optionally at least one of the carbon atoms of the cyclohexane ring bearing the substituents R¹, R^{1'}, R², R^{2'}, R³, R^{3'}, R⁴, and R^{4'} is replaced by a heteroatom, and
if the heteroatom is oxygen, the substituents R¹, R^{1'}, R², R^{2'}, R³, R^{3'}, R⁴, and R^{4'} are independently of one another selected from the group consisting of hydrogen, alkyl, alkenyl, cycloalkyl, cycloalkenyl, aryl and alkylaryl radicals.

7. Compounds according to claim 1, whereby one or two of the substituents R¹, R^{1'}, R², R^{2'}, R³, R^{3'}, R⁴, and R^{4'} are selected from the group consisting of alkyl, alkenyl, cycloalkenyl, aryl and alkylaryl radicals and the remaining radicals are hydrogen atoms.

8. Compounds according to claim 7, whereby the alkyl radicals are methyl radicals.

9. Compounds according to one of the previous claims, selected from the group consisting of
(SP-4-3-)-(4-methyl-trans-cyclohexane-1,2-diamine)oxalatoplatinum(II) and
(SP-4-3-)-(4,5-dimethyl-trans-cyclohexane-1,2-diamine)oxalatoplatinum(II).

10. Compound according to one of the previous claims as a therapeutic or prophylactic agent.

11. Use of a compound according to one of the claims 1 to 9 for the production of a medicament for combating and/or the prophylaxis of tumour diseases.

12. Medicament comprising a compound according to one of the claims 1 to 9.

## Revendications

1. Composé de formule générale (I) 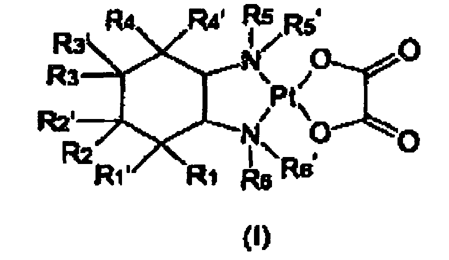 où les substituants R¹, R^{1'}, R², R^{2'}, R³, R^{3'}, R⁴ et R^{4'} sont choisis indépendamment l'un de l'autre dans le groupe formé par un atome d'hydrogène, des radicaux alkyle ramifiés ou non ramifiés, des radicaux alcényle cycloalkyle, cycloalcényle, aryle et alkylaryle ramifiés ou non ramifiés,
les substituants R⁵, R^{5'}, R⁶ et R^{6'} sont choisis indépendamment l'un de l'autre dans le groupe formé par un atome d'hydrogène, des radicaux alkyle ramifiés ou non ramifiés et des radicaux alcényle ramifiés ou non ramifiés, et
où si nécessaire à chaque fois au moins deux des substituants R¹, R^{1'}, R², R^{2'}, R³, R^{3'}, R⁴ et R^{4'} peuvent former ensemble au moins un radical alkylène, alcénylène, ou un noyau aromatique, et
où si nécessaire au moins l'un des atomes de carbone du cycle cyclohexane portant les substituants R¹, R^{1'}, R², R^{2'}, R³, R^{3'}, R⁴ et R^{4'} est remplacé par un hétéroatome, et
dans le cas où l'hétéroatome est l'oxygène, les substituants R¹, R^{1'}, R², R^{2'}, R³, R^{3'}, R⁴ et/ou R^{4'} peuvent être en outre des radicaux hydroxy, et
les sels acceptables sur le plan pharmaceutique de celui-ci,
à condition
qu'au moins un substituant R¹, R^{1'}, R², R^{2'}, R³, R^{3'}, R⁴, R^{4'} soit différent d'un atome d'hydrogène et que les substituants R¹ ou R^{1'} et R⁴ ou R^{4'} ne forment pas entre eux un radical alkylène en C₁ à C₂ non substitué.

2. Composés selon la revendication 1, dans lesquels les substituants R¹, R^{1'}, R², R^{2'}, R³, R^{3'}, R⁴ et R^{4'} sont choisis indépendamment l'un de l'autre dans le groupe formé par un atome d'hydrogène, des radicaux alkyle en C₁ à C₁₅, alcényle en C₂ à C₁₅, cycloalkyle en C₃ à C₁₅, cycloalcényle en C₃ à C₁₅, aryle en C₆ à C₁₄, et (alkyle en C₁ à C₁₅) - (aryle en C₆ à C₁₄).

3. Composés selon l'une quelconque des revendications précédentes, dans lesquels les substituants R¹, R^{1'}, R², R^{2'}, R³, R^{3'}, R⁴ et R^{4'} sont choisis dans le groupe formé par un atome d'hydrogène, des radicaux alkyle en C₁ à C₁₀, cycloalkyle en C₃ à C₁₀, et aryle en C₆ à C₁₄.

4. Composés selon l'une quelconque des revendications précédentes, dans lesquels les substituants R¹, R^{1'}, R³, R^{3'}, R⁴ et R^{4'} sont identiquement un atome d'hydrogène, et l'un des substituants R² ou R^{2'} est un radical alkyle en C₁ à C₆, ou un radical aryle en C₆ à C₁₄ et l'autre un atome d'hydrogène.

5. Composés selon la revendication 4, dans lesquels les substituants R² ou R^{2'} sont des radicaux méthyle, éthyle, propyle, t-butyle ou phényle.

6. Composés selon l'une quelconque des revendications précédentes, dans lesquels les substituants R⁵, R^{5'}, R⁶ et R^{6'} sont un atome d'hydrogène et si nécessaire au moins l'un des atomes de carbone portant les substituants R¹, R^{1'}, R², R^{2'}, R³, R^{3'}, R⁴ et R^{4'} du cycle cyclohexane est remplacé par un hétéroatome, et
dans le cas où l'hétéroatome est l'oxygène, les substituants R¹, R^{1'}, R², R^{2'}, R³, R^{3'}, R⁴ et R^{4'} sont choisis indépendamment l'un de l'autre dans le groupe formé par un atome d'hydrogène, des radicaux alkyle, alcényle, cycloalkyle, cycloalcényle, aryle et alkylaryle.

7. Composés selon la revendication 1, dans lesquels un ou deux des substituants R¹, R^{1'}, R², R^{2'}, R³, R^{3'}, R⁴ et R^{4'} sont choisis dans le groupe formé par des radicaux alkyle, alcényle, cycloalcényle, aryle et alkylaryle et les radicaux restants sont des atomes d'hydrogène.

8. Composés selon la revendication 7, dans lesquels les radicaux alkyle sont des radicaux méthyle.

9. Composés selon l'une quelconque des revendications précédentes, choisis dans le groupe formé par
le (SP-4-3-)-(4-méthyl-trans-cyclohexane-1,2-diamine)-oxalatoplatine(II) et
le (SP-4-3-)-(4,5-diméthyl-trans-cyclohexane-1,2-diamine)-oxalatoplatine(II).

10. Composé selon l'une quelconque des revendications précédentes, en tant qu'agent thérapeutique ou prophylactique.

11. Utilisation d'un composé selon l'une quelconque des revendications 1 à 9 pour préparer un médicament destiné à la lutte contre les maladies tumorales et/ou à leur prophylaxie.

12. Médicament, comprenant un composé selon l'une quelconque des revendications 1 à 9.
